# EUROPEAN PATENT SPECIFICATION

(11) **EP 3 210 485 B1**
(45) Date of publication and mention of the grant of the patent: **10.04.2019**
(21) Application number: 17157137.5
(22) Date of filing: 21.02.2017
(51) Int. Cl.: A42B 3/14

(54) **HEAD GEAR**
KOPFBEDECKUNG
COUVRE-CHEF

(30) Priority: 23.02.2016 US 201615051043
(43) Date of publication of application: 30.08.2017
(73) Proprietor: A.C.E. International, Taunton, MA 02780 (US)
(72) Inventor: PEREIRA, Jason M., Taunton, MA Massachusetts 02780 (US); WATKINS, James, East Taunton, MA Massachusetts 02718 (US); MARTIN, Ed, Plymouth, MA Massachusetts 02360 (US)
(74) Representative: Zinkler, Franz

(56) References cited:
- US-A- 2 763 006
- US-A1- 2015 250 251
- US-A1- 2015 359 286

## Description

### BACKGROUND

### FIELD OF THE DISCLOSURE

The present disclosure relates generally to safety equipment and, more particularly, to head gear.

### DESCRIPTION OF RELATED ART

Certain work environments require protective gear for the head and face (e.g., welding helmet, brow guard, etc.). Sometimes, the protective gear is cumbersome and uncomfortable. Furthermore, the protective gear may not be an optimal fit for all users, thereby further adding to the discomfort. Given the importance of this type of safety equipment, there are ongoing efforts to improve the designs associated with protective gear. US 2015/359286 discloses an adapter for a protective head gear comprising a clip with a groove, and a rack that engages with the groove, wherein the forehead strap comprises upper and lower portions which pivot jointly about a left and right pivot point.

### SUMMARY

The present invention is defined in independent claim 1.

A protective head gear comprises a lower front strap, an upper front strap, a right side strap, and a left side strap. The right side strap comprises a right pivot point. The left side strap comprises a left pivot point. The lower front strap comprises a lower left attachment point that is pivotally attached to the left pivot point on the left side strap. The upper front strap comprises an upper left attachment point that is also pivotally attached to the left pivot point on the left side strap. The lower front strap comprises a lower right attachment point that is pivotally attached to the right pivot point on the right side strap. The upper front strap comprises an upper right attachment point that is also pivotally attached to the right pivot point on the right side strap.

Other features, and advantages will be or become apparent to one with skill in the art upon examination of the following drawings and detailed description. It is intended that all such additional features, and advantages be included within this description, be within the scope of the present disclosure, and be protected by the accompanying claims.

### BRIEF DESCRIPTION OF THE DRAWINGS

Many aspects of the disclosure can be better understood with reference to the following drawings. The components in the drawings are not necessarily to scale, emphasis instead being placed upon clearly illustrating the principles of the present disclosure. Moreover, in the drawings, like reference numerals designate corresponding parts throughout the several views.
FIG. 1 is a diagram showing one embodiment of a protective head gear.
FIGS. 2A and 2B (collectively, FIG. 2) are diagrams showing one embodiment of an exploded view (FIG. 2A) and a front view (FIG. 2B) of a frontal strap attachment for the protective head gear of FIG. 1.
FIG. 3A is a diagram showing one embodiment of a right side of a protective head gear without a rack lock.
FIG. 3B is a diagram showing one embodiment of a left side of a protective head gear without a rack lock.
FIG. 4A is a diagram showing one embodiment of a left side of a protective head gear with a paddle lock.
FIG. 4B is a diagram showing one embodiment of a right side of a protective head gear with a paddle lock.
FIG. 5A is a diagram showing one embodiment of a left side of a protective head gear with a push-button lock.
FIG. 5B is a diagram showing one embodiment of a right side of a protective head gear with a push-button lock.
FIGS. 6A, 6B, and 6C (collectively, FIG. 6) are diagrams showing a front view (FIG. 6A), a side view (FIG. 6B), and a rear view (FIG. 6C) (collectively, orthogonal views) of one embodiment of a busing or bearing.
FIGS. 7A, 7B, and 7C (collectively, FIG. 7) are diagrams showing orthogonal views of one embodiment of a dial with integrated pinion (or pinioned dial).
FIGS. 8A, 8B, 8C, 8D, 8E, and 8F (collectively, FIG. 8) are diagrams showing orthogonal views of one embodiment of a rack with pawl, which can also act as a horizontal position indicator pin, at the top of the rack.
FIGS. 9A1, 9A2, 9A3, 9A4, 9A5, and 9A6 (collectively, FIG. 9A) are diagrams showing orthogonal views of one embodiment of a right slide lock.
FIGS. 9B1, 9B2, 9B3, 9B4, 9B5, and 9B6 (collectively, FIG. 9B) are diagrams showing orthogonal views of one embodiment of a left slide lock.
FIGS. 10A1, 10A2, 10A3, 10A4, 10A5, and 10A6 (collectively, FIG. 10A) are diagrams showing orthogonal views of one embodiment of a right cover.
FIGS. 10B1, 10B2, 10B3, 10B4, 10B5, and 10B6 (collectively, FIG. 10B) are diagrams showing orthogonal views of one embodiment of a left cover.
FIGS. 11A1, 11A2, 11A3, 11A4, 11A5, and 11A6 (collectively, FIG. 11A) are diagrams showing orthogonal views of another embodiment of a right cover.
FIGS. 11B1, 11B2, 11B3, 11B4, 11B5, and 11B6 (collectively, FIG. 11B) are diagrams showing orthogonal views of another embodiment of a left cover.
FIGS. 12A, 12B, 12C, and 12D (collectively, FIG. 12) are diagrams showing orthogonal views of one embodiment of a top rotation strap connector.
FIGS. 13A, 13B, and 13C (collectively, FIG. 13) are diagrams showing orthogonal views of one embodiment of a lower front strap.
FIGS. 14A, 14B, and 14C (collectively, FIG. 14) are diagrams showing orthogonal views of one embodiment of an upper front strap.
FIGS. 15A1, 15A2, 15A3, 15A4, and 15A5 (collectively FIG. 15A) are diagrams showing orthogonal views of one embodiment of a right side strap.
FIGS. 15B1, 15B2, 15B3, 15B4, and 15B5 (collectively FIG. 15B) are diagrams showing orthogonal views of one embodiment of a left side strap.
FIGS. 16A1, 16A2, 16A3, 16A4, 16A5, and 16A6 (collectively FIG. 16A) are diagrams showing orthogonal views of one embodiment of a right mount for use without a rack lock.
FIGS. 16B1, 16B2, 16B3, 16B4, 16B5, and 16B6 (collectively FIG. 16B) are diagrams showing orthogonal views of one embodiment of a left mount for use without a rack lock.
FIGS. 17A1, 17A2, 17A3, 17A4, 17A5, and 17A6 (collectively FIG. 17A) are diagrams showing orthogonal views of one embodiment of a right mount for use with a paddle lock.
FIGS. 17B1, 17B2, 17B3, 17B4, 17B5, and 17B6 (collectively FIG. 17B) are diagrams showing orthogonal views of one embodiment of a left mount for use with a paddle lock.
FIGS. 18A1, 18A2, 18A3, 18A4, 18A5, and 18A6 (collectively FIG. 18A) are diagrams showing orthogonal views of one embodiment of a right mount for use with a push-button lock.
FIGS. 18B1, 18B2, 18B3, 18B4, 18B5, and 18B6 (collectively FIG. 18B) are diagrams showing orthogonal views of one embodiment of a left mount for use with a push-button lock.
FIGS. 19A, 19B, 19C, and 19D (collectively, FIG. 19) are diagrams showing orthogonal views of one embodiment of a push button.
FIGS. 20A, 20B, 20C, and 20D (collectively, FIG. 20) are diagrams showing orthogonal views of one embodiment of a paddle.
FIG. 21 is a diagram showing another embodiment of a protective head gear.
FIG. 22A is a diagram showing another embodiment of a right side of a protective head gear without a rack lock.
FIG. 22B is a diagram showing another embodiment of a left side of a protective head gear without a rack lock.
FIG. 23A is a diagram showing another embodiment of a right side of a protective head gear with a paddle lock.
FIG. 23B is a diagram showing another embodiment of a left side of a protective head gear with a paddle lock.
FIG. 24A is a diagram showing another embodiment of a right side of a protective head gear with a push-button lock.
FIG. 24B is a diagram showing another embodiment of a left side of a protective head gear with a push-button lock.
FIGS. 25A, 25B, 25C, 25D, 25E, and 25F (collectively, FIG. 25) are diagrams showing orthogonal views of one embodiment of a right inner clip.
FIGS. 26A, 26B, 26C, 26D, 26E, and 26F (collectively, FIG. 26) are diagrams showing orthogonal views of one embodiment of a left inner clip.
FIGS. 27A, 27B, 27C, 27D, 27E, and 27F (collectively, FIG. 27) are diagrams showing orthogonal views of one embodiment of a right outer clip for use without a rack lock.
FIGS. 28A, 28B, 28C, 28D, 28E, and 28F (collectively, FIG. 28) are diagrams showing orthogonal views of one embodiment of a left outer clip for use without a rack lock.
FIGS. 29A, 29B, 29C, 29D, 29E, and 29F (collectively, FIG. 29) are diagrams showing orthogonal views of one embodiment of a right outer clip for use with a push-button lock.
FIGS. 30A, 30B, 30C, 30D, 30E, and 30F (collectively, FIG. 30) are diagrams showing orthogonal views of one embodiment of a left outer clip for use with a push-button lock.
FIGS. 31A, 31B, 31C, 31D, 31E, and 31F (collectively, FIG. 31) are diagrams showing orthogonal views of one embodiment of a right outer clip for use with a paddle lock.
FIGS. 32A, 32B, 32C, 32D, 32E, and 32F (collectively, FIG. 32) are diagrams showing orthogonal views of one embodiment of a left outer clip for use with a paddle lock.
FIGS. 33A, 33B, 33C, 33D, and 33E (collectively, FIG. 33) are diagrams showing orthogonal views of one embodiment of a back strap connector (labeling of left shown in FIG. 33B; labeling of right shown on FIG. 33D).
FIG. 34A is a diagram showing a right side of another embodiment of a protective head gear.
FIG. 34B is a diagram showing a left side of another embodiment of a protective head gear.
FIGS. 35A, 35B, 35C, 35D, 35E, and 35F (collectively, FIG. 35) are diagrams showing orthogonal views of one embodiment of a right side inner clip for use with a spring clip.
FIGS. 36A, 36B, 36C, 36D, 36E, and 36F (collectively, FIG. 36) are diagrams showing orthogonal views of one embodiment of a right side outer clip for use with a spring clip.
FIGS. 37A, 37B, 37C, 37D, and 37E (collectively, FIG. 37) are diagrams showing orthogonal views of one embodiment of a spring clip.
FIGS. 38A and 38B (collectively, FIG. 38) are diagrams showing orthogonal views of one embodiment of a shaft pin for a hinge.
FIGS. 39A, 39B, 39C, 39D, 39E and 39F (collectively, FIG. 39) are diagrams showing orthogonal views of one embodiment of a left side inner clip for use with a spring clip.
FIGS. 40A, 40B, 40C, 40D, 40E and 40F (collectively, FIG. 40) are diagrams showing orthogonal views of one embodiment of a left side outer clip for use with a spring clip.

### DETAILED DESCRIPTION OF THE EMBODIMENTS

Various hazardous working environments require the use of protective gear for the head and face (e.g., welding helmet, brow guard, etc.). Depending on the work, the protective gear can sometimes be cumbersome and uncomfortable. Sometimes, the discomfort associated with protective gear becomes a deterrent for the operator to wear the protective gear. In some situations, an uncomfortable fit can also be dangerous, since the discomfort can become a distraction. Prior solutions have included adapters for protective head gear, such as those found in U.S. Patent Publication Numbers US-2015-0143618-A1 and US-2015 -0143669-A1, both published on 2015 May 28, both having the title "Adapter for Protective Head Gear", by Pereira et al. (hereafter "Pereira system" or "Pereira device").

Although the Pereira device provides a more comfortable fit for protective gear that what was previously known, better comfort and fit can be achieved. For example, as shown herein, instead of using a single front strap (as in the Pereira device), the disclosed system employs an additional front strap, thereby providing greater security when using a brow guard, welding helmet, or other protective gear. In other embodiments, a locking mechanism is provided, thereby reducing the likelihood of relative movement between a mount and a rack. Adding another front strap and providing locking mechanisms (such as, for example, a paddle lock or a push-button lock) results in greater comfort and improved safety.

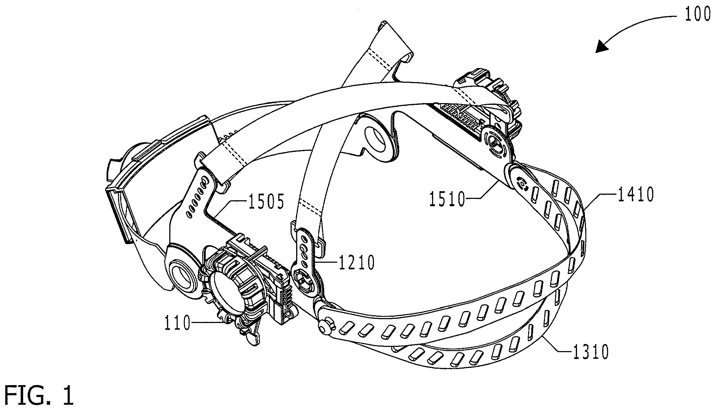

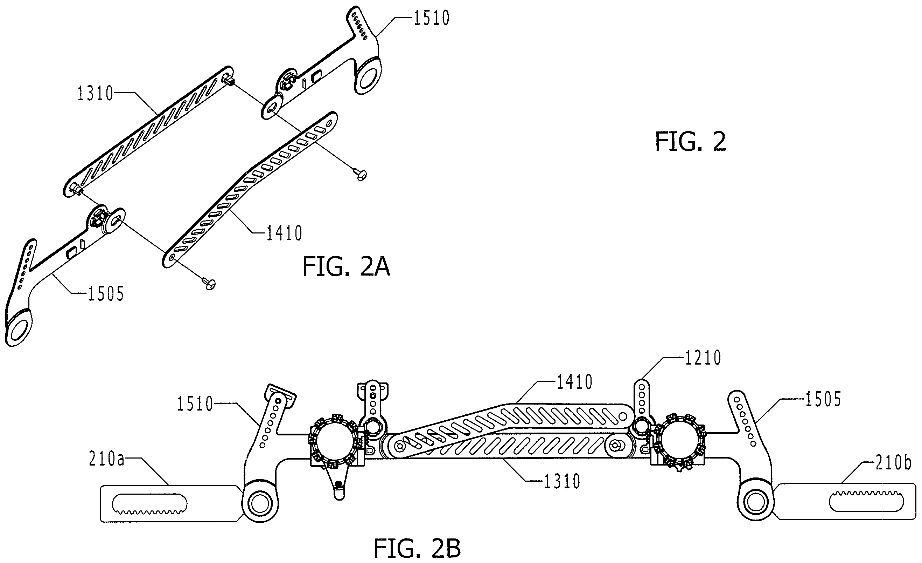

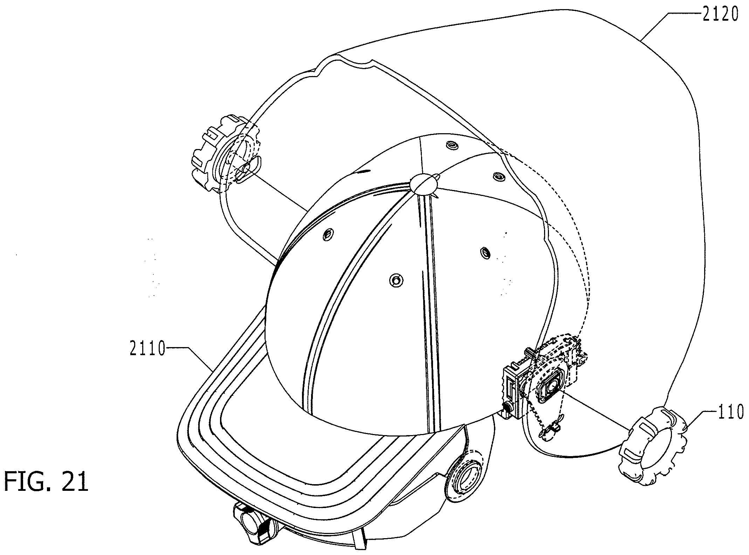

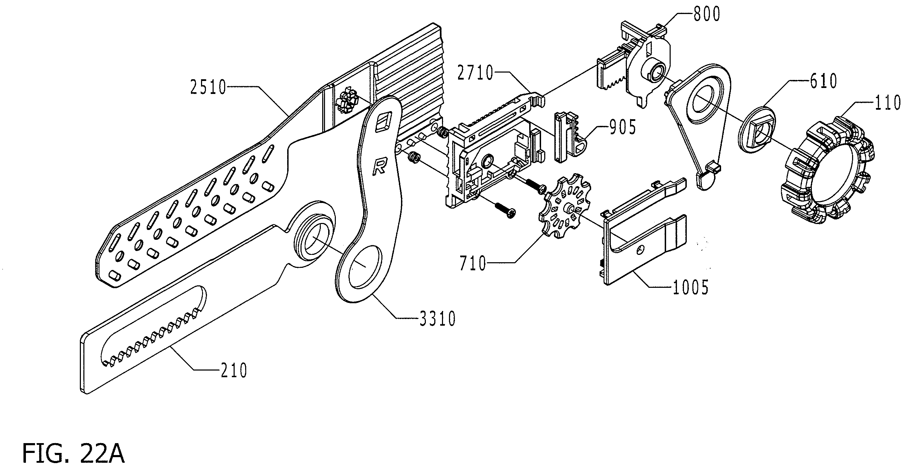

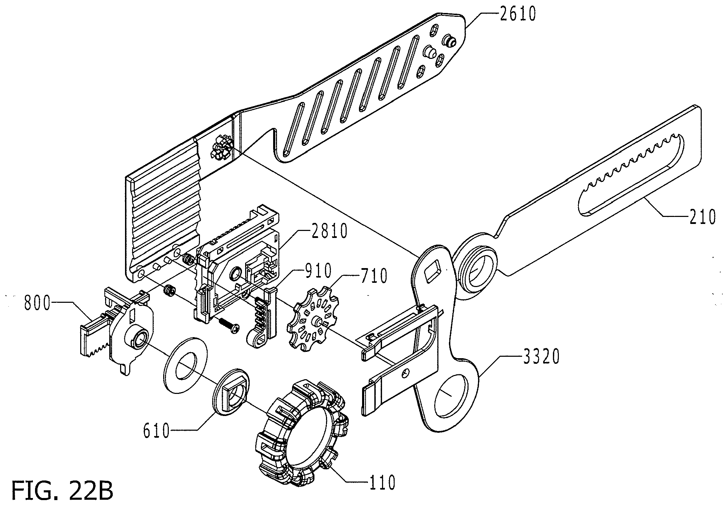

Having provided a general overview of the benefits of the disclosed apparatus and system, reference is now made in detail to the description of the embodiments as illustrated in the drawings. While several embodiments are described in connection with these drawings, there is no intent to limit the disclosure to the embodiment or embodiments disclosed herein. On the contrary, the intent is to cover all alternatives, modifications, and equivalents. For clarity, one embodiment of protective head gear is described with reference to FIGS. 1 through 20, with components of different embodiments shown in FIGS. 2 through 20. Thereafter, another embodiment of protective head gear (e.g., for use with a baseball cap or other flexible non-rigid head covering) is described with reference to FIGS. 21 through 33, with components of different embodiments shown in FIGS. 22 through 33.

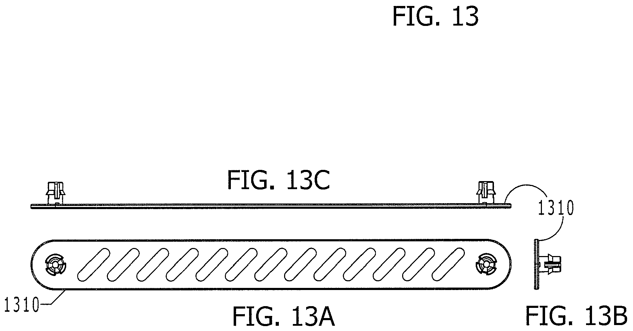

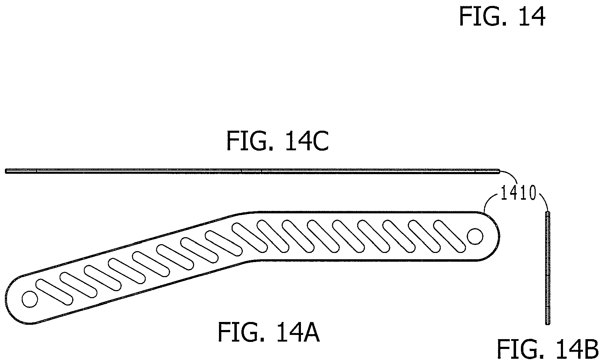

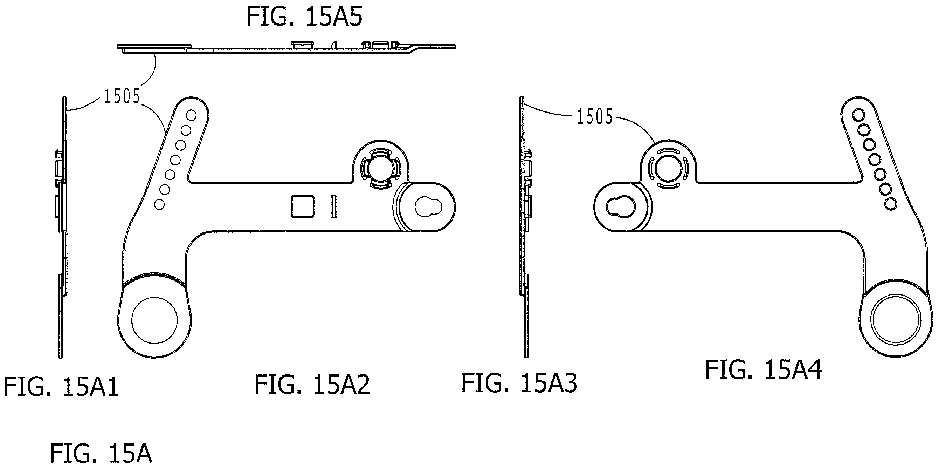

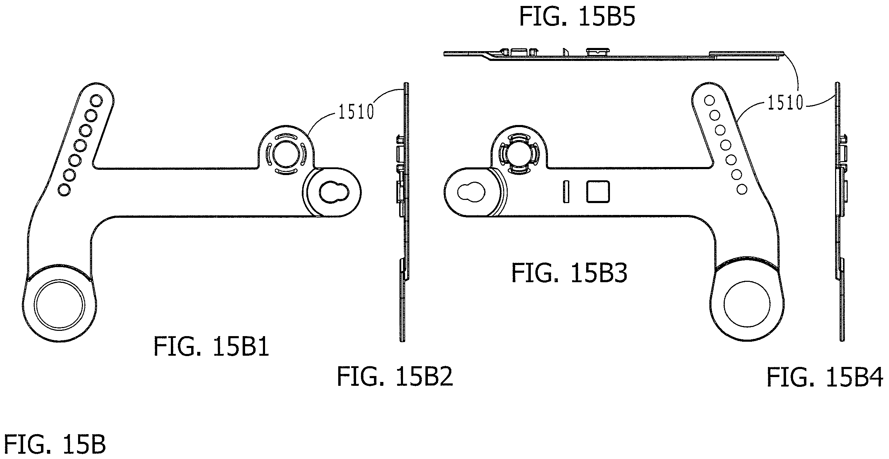

With this in mind, attention is turned to FIG. 1, which is a diagram showing one embodiment of a protective head gear 100. In the embodiment of FIG. 1, the primary components of the protective head gear 100 include a left side strap 1510, a right side strap 1505, a lower front strap 1310, and an upper side strap 1410. Orthogonal views of the lower front strap 1310 are shown in detail in FIGS. 13A, 13B, and 13C (collectively, FIG. 13); orthogonal views of the upper front strap 1410 are shown in detail in FIGS. 14A, 14B, and 14C (collectively, FIG. 14); orthogonal views of the right side strap 1505 are shown in FIGS. 15A1, 15A2, 15A3, 15A4, and 15A5 (collectively, FIG. 15A); and orthogonal views of the left side strap 1510 are shown in FIGS. 15B1, 15B2, 15B3, 15B4, and 15B5 (collectively, 15B).

As shown in FIGS. 1, 13, and 15B, the lower front strap 1310 comprises a left attachment point (for convenience, this attachment point is designated as a lower left attachment point). The lower left attachment point is pivotally connected to the left side strap 1510 at pivot point located on the left side strap 1510 (for convenience, the pivot point on the left side strap 1510 is designated as the left pivot point). The pivotal connection between the lower front strap 1310 and the left side strap 1510 permits angular movement of the lower front strap 1310 with reference to the left side strap 1510.

Next, as shown in FIGS. 1, 13, and 15A, the lower front strap 1310 also comprises a right attachment point (designated as lower right attachment point). The lower right attachment point is pivotally connected to the right side strap 1505 at a pivot point located on the right side strap 1510 (for convenience, the pivot point on the right side strap 1510 is designated as the right pivot point). Similar to the left side, the pivotal connection between the lower front strap 1310 and the right side strap 1505 permits angular movement of the lower front strap 1310 with reference to the right side strap 1505.

In addition to the lower front strap 1310, the upper front strap 1410 is similarly connected to the left side strap 1510 and the right side strap 1505. Specifically, as shown in FIGS. 1, 14, and 15B, the upper front strap 1410 comprises a left attachment point (designated as an upper left attachment point). The upper left attachment point is pivotally connected to the left side strap 1510 at the same left pivot point to which the lower front strap 1310 is pivotally connected. Again, the pivotal connection between the upper front strap 1410 and the left side strap 1510 permits angular movement of the upper front strap 1410 with reference to the left side strap 1510.

Next, as shown in FIGS. 1, 14, and 15A, the upper front strap 1410 also comprises a right attachment point (designated as upper right attachment point). The upper right attachment point is pivotally connected to the right side strap 1505 at the same right pivot point to which the lower right attachment point is pivotally connected. Again, the pivotal connection between the upper front strap 1410 and the right side strap 1505 permits angular movement of the upper front strap 1410 with reference to the right side strap 1505.

Additionally, by permitting independent movement of the lower front strap 1310 and the upper front strap 1410, the two front straps 1310, 1410 can be positioned independently at different angles to provide greater comfort, better fit, and greater security. Consequently, the two front straps 1310, 1410 on the protective head gear 100 provides improved safety compared to a single strap.

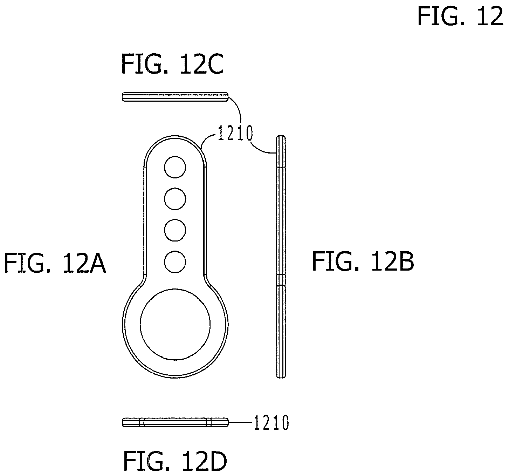

FIG. 1 also shows a top rotation strap connector 1210 that is pivotally connected to each of the side straps 1505, 1510. Orthogonal views of the top rotation strap connector 1210 are shown in detail in FIGS. 12A, 12B, 12C, and 12D (collectively FIG. 12). Additionally, FIG. 1 shows a knob 110 that secures a brow guard, welding mask, etc., to the protective head gear 100. For clarity, FIGS. 2A and 2B (collectively, FIG. 2) are diagrams showing one embodiment of an exploded view (FIG. 2A) and a front view (FIG. 2B) of a frontal strap attachment for the protective head gear of FIG. 1. As shown in the front view (FIG. 2B), a left back strap 210b is pivotally coupled to the back of the left side strap 1505 and a right back strap 210a is pivotally coupled to the back of the right side strap 1510. Insofar as similar back straps have been described in detail with reference to the Pereira system in the published patents, referenced above, further discussion of the back straps is omitted here.

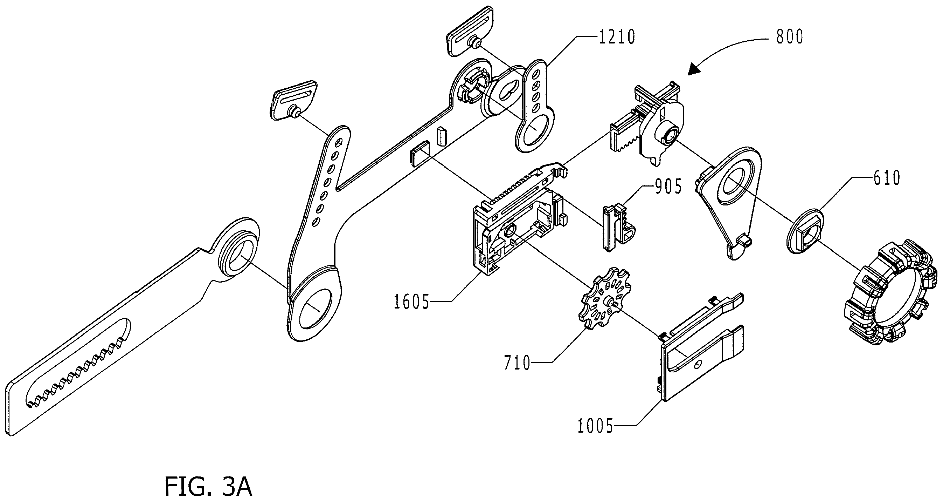

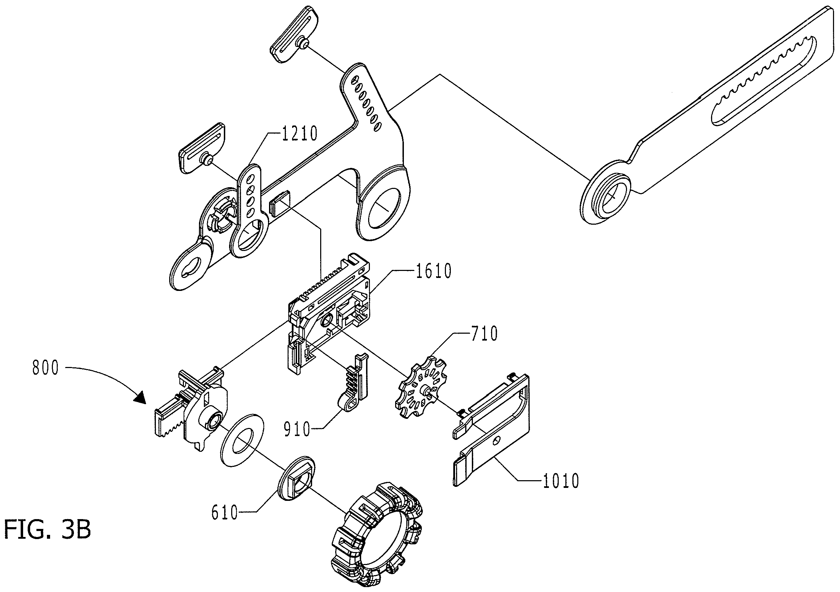

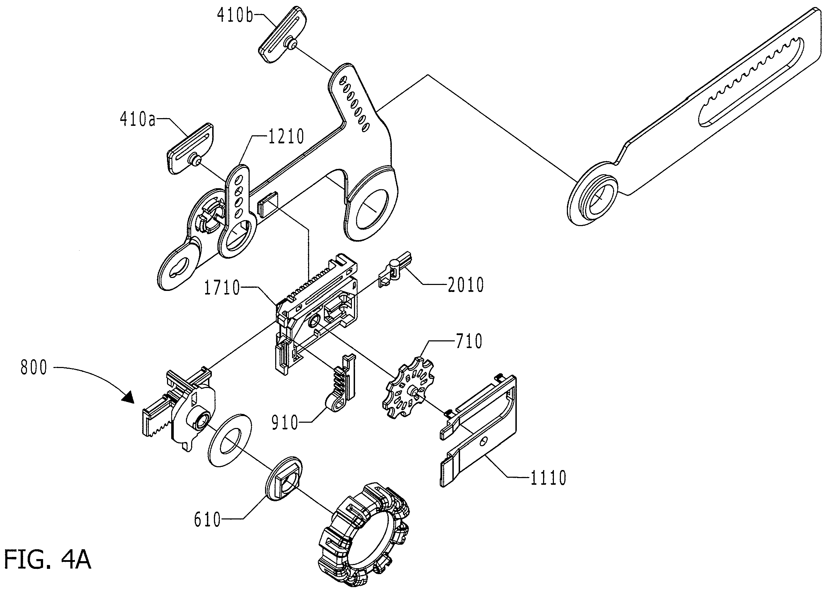

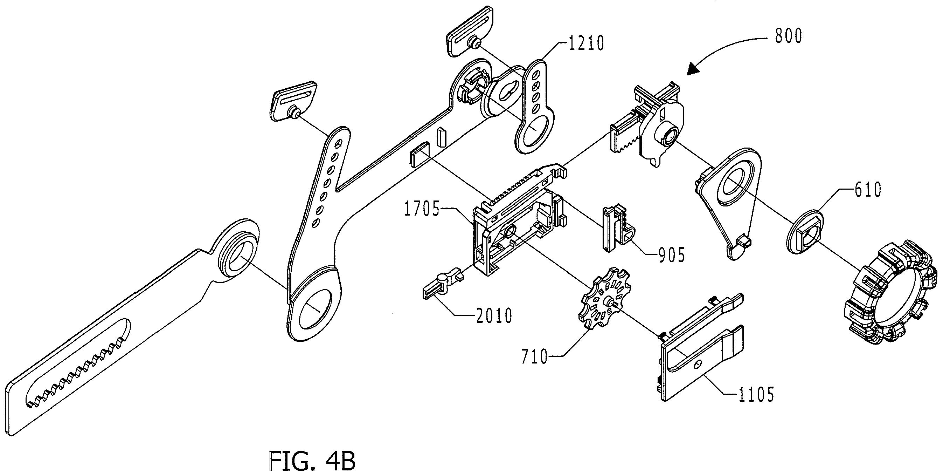

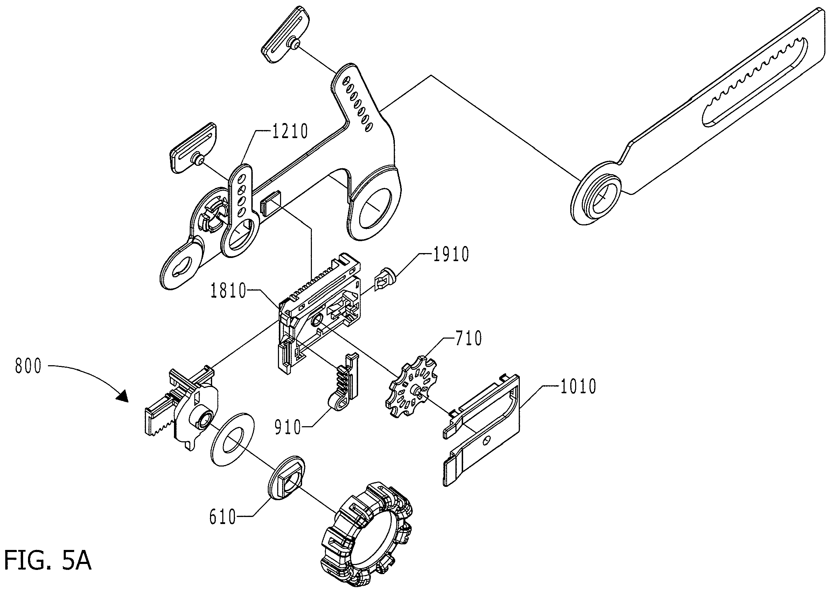

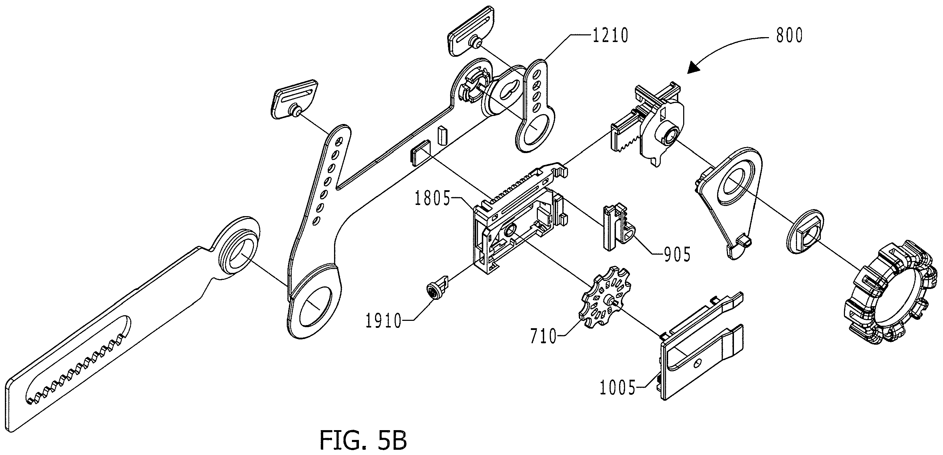

In addition to a dual-frontal-strap system, as shown in FIGS. 1 and 2, further security is possible by providing a selective locking mechanism. For comparison, a system with no locking mechanism is shown with reference to FIGS. 3A and 3B (collectively, FIG. 3), while a paddle lock mechanism is shown in FIGS. 4A and 4B (collectively, FIG. 4) and a push-button lock mechanism is shown in FIGS. 5A and 5B (collectively, FIG. 5).

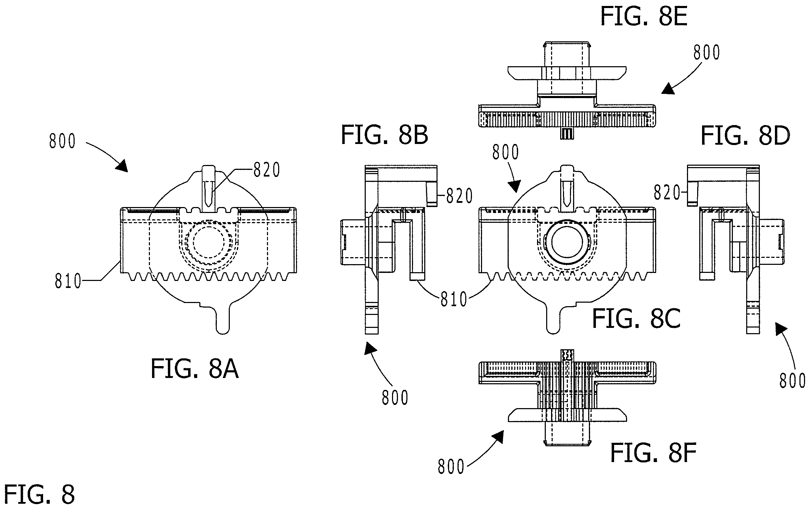

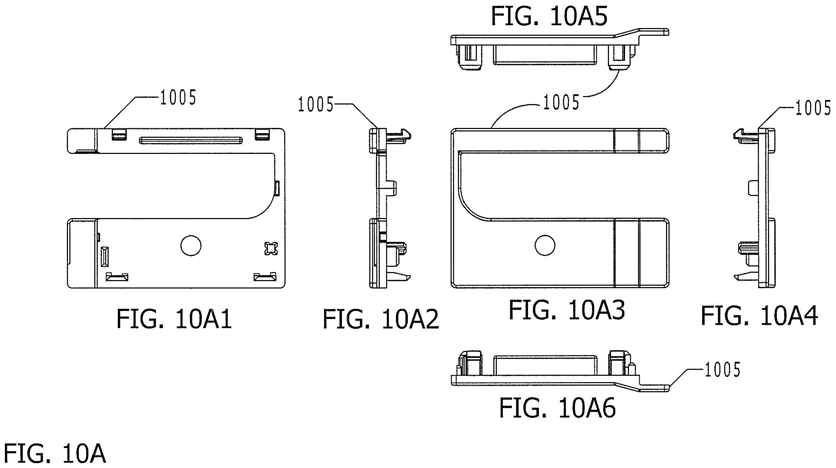

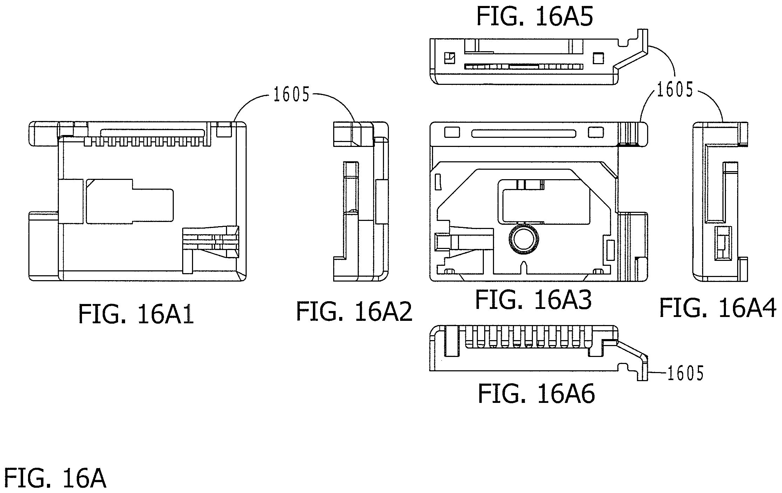

As shown in FIG. 3A, the right side of an apparatus without a locking mechanism comprises a right mount 1605 that is mechanically coupled to the right side strap 1505, a rack 800 that is slidably coupled to the right mount 1605, and a right cover 1005 that is mechanically coupled (meaning, affixed by whatever means) to the right mount 1605 such that the rack 800 is located between the right mount 1605 and the right cover 1005. Orthogonal views of the rack 800, along with its teeth 810 and pawl 820, which can also act as a horizontal position indicator pin, are shown in FIGS. 8A, 8B, 8C, 8D, 8E, and 8F (collectively, FIG. 8); orthogonal views of the right mount 1605 are shown in FIGS. 16A1, 16A2, 16A3, 16A4, and 16A5 (collectively FIG. 16A); and orthogonal views of the right cover 1005 are shown in FIGS. 10A1, 10A2, 10A3, 10A4, 10A5, and 10A6 (collectively, FIG. 10A).

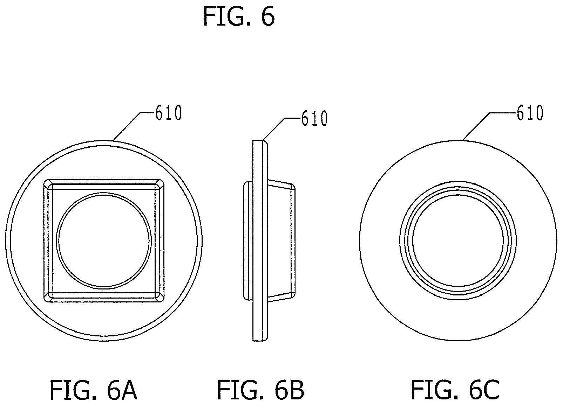

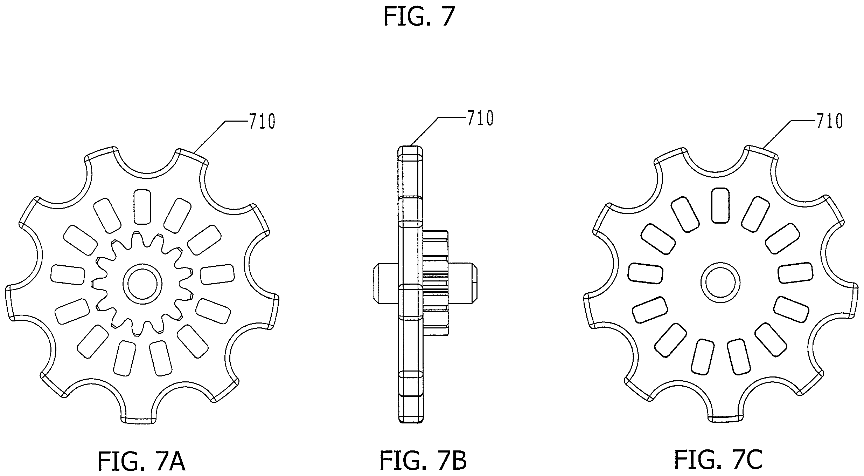

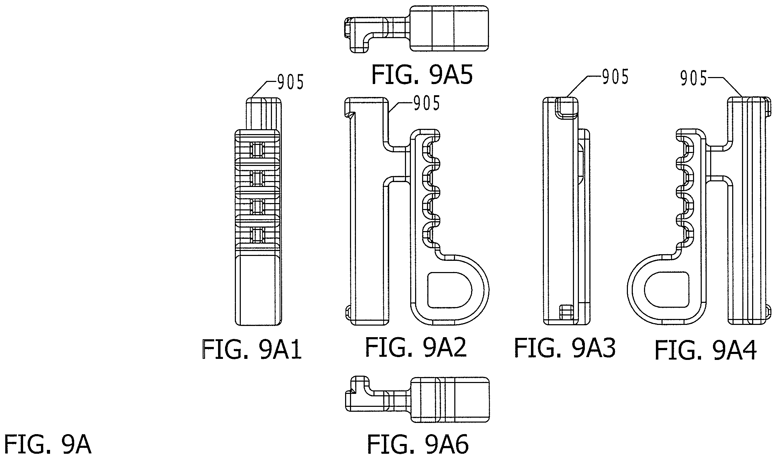

Continuing with FIG. 3A, a right slide lock 905 and a pinioned dial 710 are both located between the right mount 1605 and the right cover 1005. Specifically, the pinioned dial 710 is rotationally coupled to the right mount 1605 and positioned in such a way as to permit a rack-and-pinion movement between the pinioned dial 710 and the teeth 810 of the rack 800. In other words, turning the pinioned dial 710 results in a sliding motion of the rack 810. FIG. 3A also shows a bushing 610 that is secured with the knob 110, and which secures in place a mechanism that permits angular adjustment of a brow guard (not shown), a welding helmet (not shown), or other protective face masks (not shown). The right slide lock 905 either allows or disallows removal of a protective device by opening a channel in which the rack 800 slides so that the rack 800 may slide out of the mount with the protective device still attached. Orthogonal views of the right slide lock 905 are shown in FIGS. 9A1, 9A2, 9A3, 9A4, 9A5, and 9A6 (collectively, FIG. 9A); orthogonal views of the pinioned dial 710 are shown in FIGS. 7A, 7B, and 7C (collectively, FIG. 7); and orthogonal views of the bushing 610 are shown in FIGS. 6A, 6B, and 6C (collectively, FIG. 6). Insofar as the knob 110 is described with reference to the Pereira system, and insofar as the function of the bushing 610 can be readily discerned by those having skill in the art, further discussion of the knob and 110 the bushing 610 is omitted from the remainder of this disclosure.

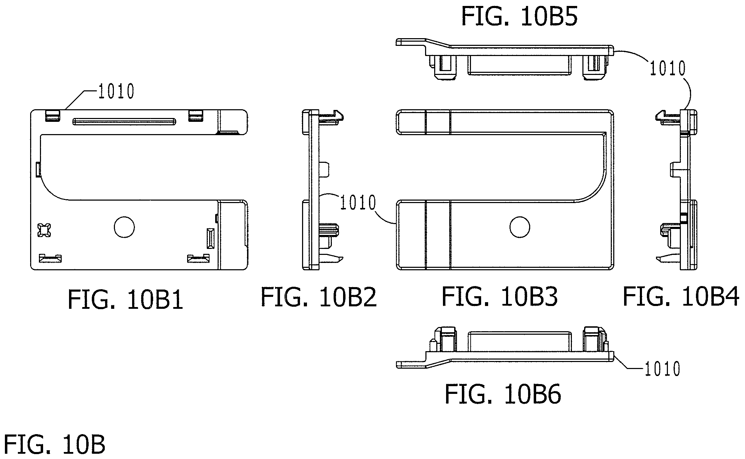

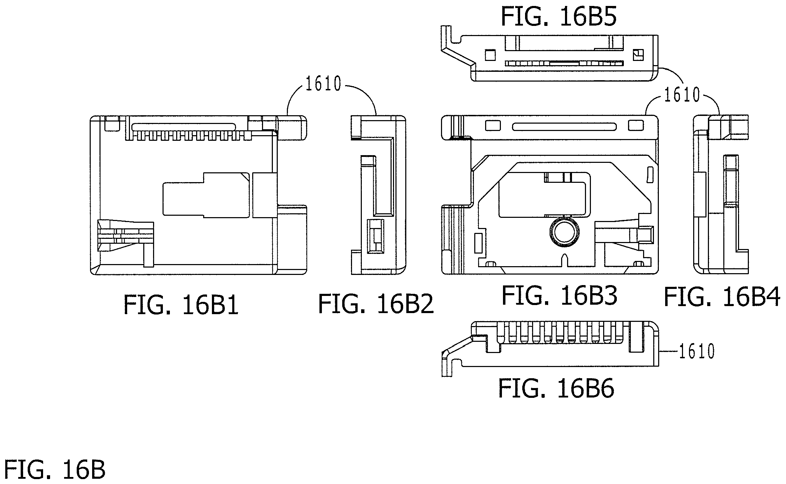

Corresponding to FIG. 3A (which shows the right side of the apparatus without a locking mechanism), the left side of the apparatus without a locking mechanism is shown in FIG. 3B. As shown in FIG. 3B, the left side comprises a left mount 1610 that is mechanically coupled to the left side strap 1510, a rack 800 that is slidably coupled to the left mount 1610, and a left cover 1010 that is affixed (or mechanically coupled) to the left mount 1610 such that the rack 800 is located between the left mount 1610 and the left cover 1010. Orthogonal views of the left mount 1610 are shown in FIGS. 16B1, 16B2, 16B3, 16B4, and 16B5 (collectively FIG. 16B); and orthogonal views of the left cover 1010 are shown in FIGS. 10B1, 10B2, 10B3, 10B4, 10B5, and 10B6 (collectively, FIG. 10B).

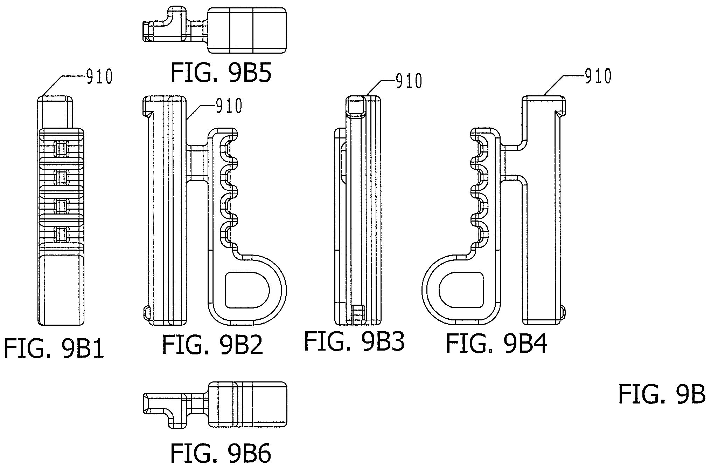

Similar to FIG. 3A, in FIG. 3B a left slide lock 910 and a pinioned dial 710 are located between the left mount 1610 and the left cover 1010. Again, the pinioned dial 710 is rotationally coupled to the left mount 1610 and positioned in such a way as to permit a rack-and-pinion movement between the pinioned dial 710 and the teeth 810 of the rack 800. The left slide lock 910 (similar to the right slide lock 905) allows or disallows removal of a protective device by opening a channel in which the rack 800 slides so that the rack 800 may slide out of the mount with the protective device still attached. Orthogonal views of the left slide lock 910 are shown in FIGS. 9B1, 9B2, 9B3, 9B4, 9B5, and 9B6 (collectively, FIG. 9B).

Having described one embodiment of an apparatus without a locking mechanism, attention is turned to FIGS. 4A and 4B, which show one embodiment of an apparatus with a paddle locking mechanism.

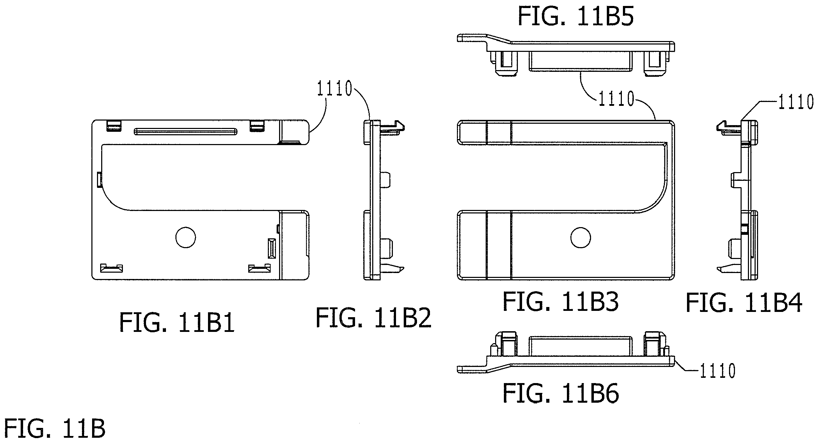

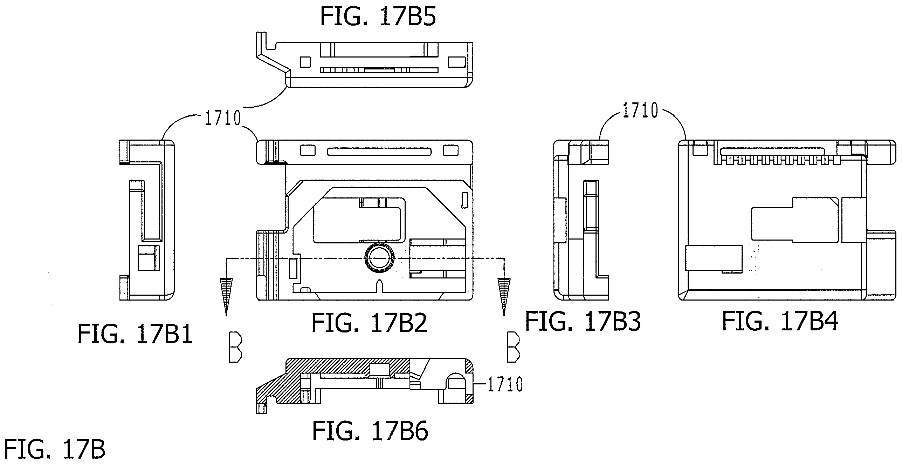

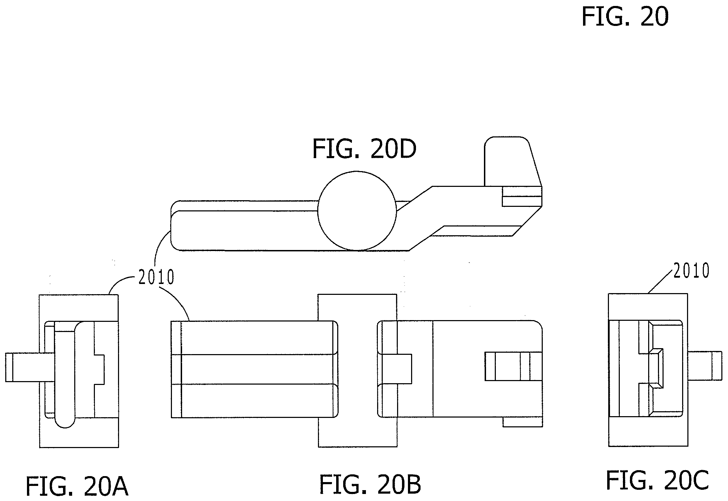

As shown in FIG. 4A, the left side of an apparatus with a paddle locking mechanism comprises a left mount 1710 that is mechanically coupled to the left side strap 1510, a rack 800 that is slidably coupled to the left mount 1710, and a left cover 1110 that is mechanically coupled to the left mount 1710. The rack 800 is located between the left mount 1710 and the left cover 1110. Additionally, FIG. 4A shows a paddle 2010 (also called a rocker) that can be manually adjusted to lock the dial which, in turn, prevents movement of the rack 800 to prevent the rack 800 from sliding, thereby improving security of the protective head gear. Orthogonal views of the left mount 1710 are shown in FIGS. 17B1, 17B2, 17B3, 17B4, and 17B5 (collectively FIG. 17B); orthogonal views of the paddle 2010 are shown in FIGS. 20A, 20B, 20C, and 20D (collectively, FIG. 20); and orthogonal views of the left cover 1110 are shown in FIGS. 11B1, 11B2, 11B3, 11B4, 11B5, and 11B6 (collectively, FIG. 11B).

A left slide lock 910 and a pinioned dial 710 are both located between the left mount 1710 and the left cover 1110, with the pinioned dial 710 being rotationally coupled to the left mount 1710 so as to establish a rack-and-pinion mechanism with the rack 800. The paddle 2010, when locked, prevents the dial 710 from moving, thereby also preventing the rack 800 from moving. Also shown in FIG. 4A are top-strap attachments 410a, 410b. Insofar as top-strap attachments are described with reference to the Pereira device, further discussion of the top-strap attachments is omitted throughout the remainder of this disclosure.

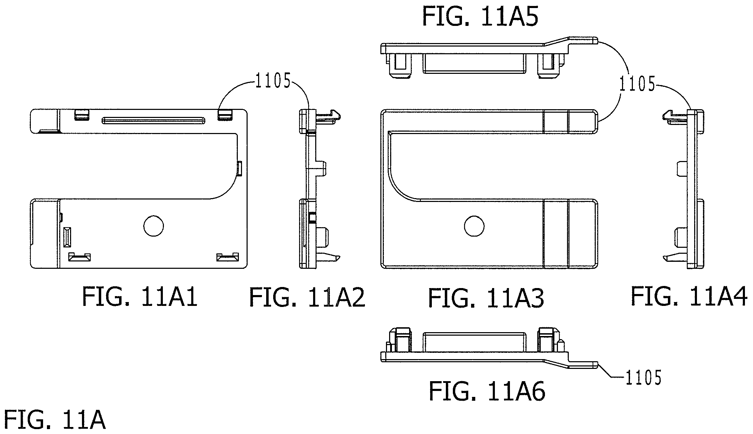

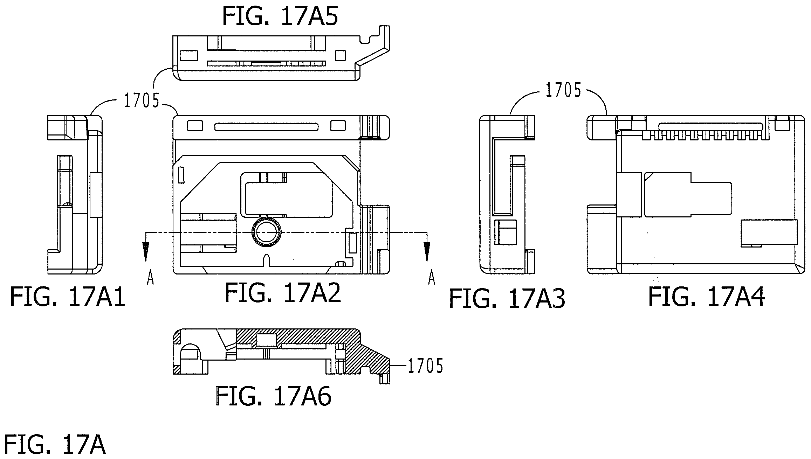

Corresponding to FIG. 4A (which shows the left side of the apparatus with a paddle locking mechanism), the right side of the apparatus with a paddle locking mechanism is shown in FIG. 4B. As shown in FIG. 4B, the right side comprises a right mount 1705 that is mechanically coupled to the right side strap 1505, a rack 800 that is slidably coupled to the right mount 1705, and a right cover 1105 that is affixed to the right mount 1705 such that the rack 800 is located between the right mount 1705 and the right cover 1105. Orthogonal views of the right mount 1705 are shown in FIGS. 17A1, 17A2, 17A3, 17A4, and 17A5 (collectively FIG. 17A); and orthogonal views of the right cover 1105 are shown in FIGS. 11A1, 11A2, 11A3, 11A4, 11A5, and 11A6 (collectively, FIG. 11A).

Similar to FIG. 4A, in FIG. 4B a right slide lock 905 and a pinioned dial 710 are located between the right mount 1705 and the right cover 1105. Again, the pinioned dial 710 is rotationally coupled to the right mount 1705 and positioned in such a way as to permit a rack-and-pinion movement between the pinioned dial 710 and the rack 800. Similar to FIG. 4A, the paddle 2010, when locked, prevents movement of the dial 710, thereby preventing the rack 800 from moving.

Turning to FIGS. 5A and 5B, a push-button locking mechanism is described.

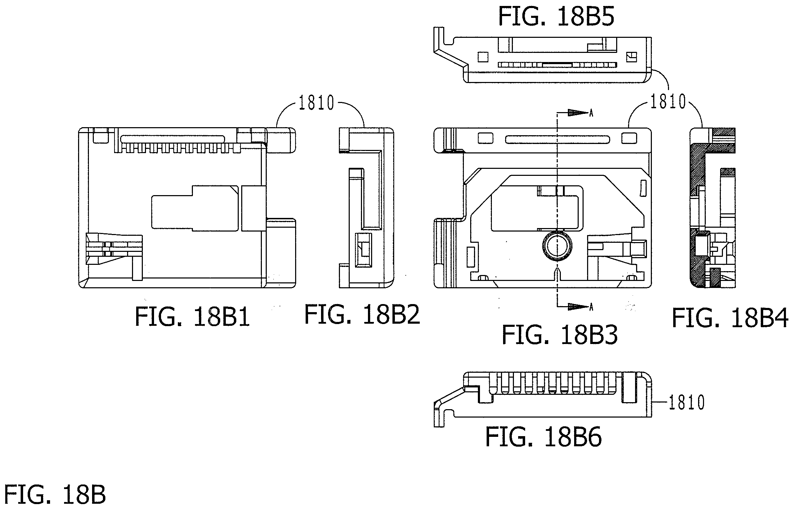

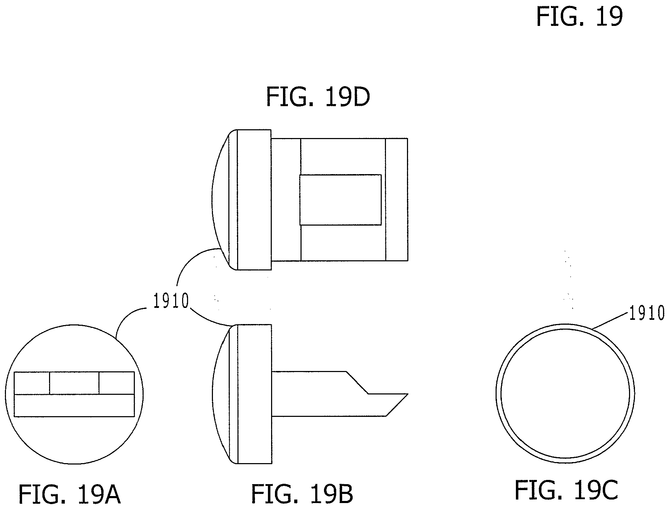

As shown in FIG. 5A, the left side of an apparatus with a push-button locking mechanism comprises a left mount 1810 that is mechanically coupled to the left side strap 1510, a rack 800 that is slidably coupled to the left mount 1810, and a left cover 1010 that is mechanically coupled to the left mount 1810. It should be appreciated that the left cover 1010 in this embodiment can be the same left cover 1010 that is used for the embodiment that has no locking mechanism. In other words, the two left covers 1010 are interchangeable for these particular embodiments. The rack 800 is located between the left mount 1810 and the left cover 1010. FIG. 5A also shows a push button 1910 that can be manually toggled to lock the rack 800 to prevent the rack 800 from sliding. Orthogonal views of the left mount 1810 are shown in FIGS. 18B1, 18B2, 18B3, 18B4, and 18B5 (collectively FIG. 18B); and orthogonal views of the push button 1910 are shown in FIGS. 19A, 19B, 19C, and 19D (collectively, FIG. 19). A left slide lock 910 and a pinioned dial 710 are both located between the left mount 1810 and the left cover 1010, with the pinioned dial 710 being rotationally coupled to the left mount 1810 so as to establish a rack-and-pinion mechanism with the rack 800. The push button 1910, when locked, prevents the rack 800 from sliding.

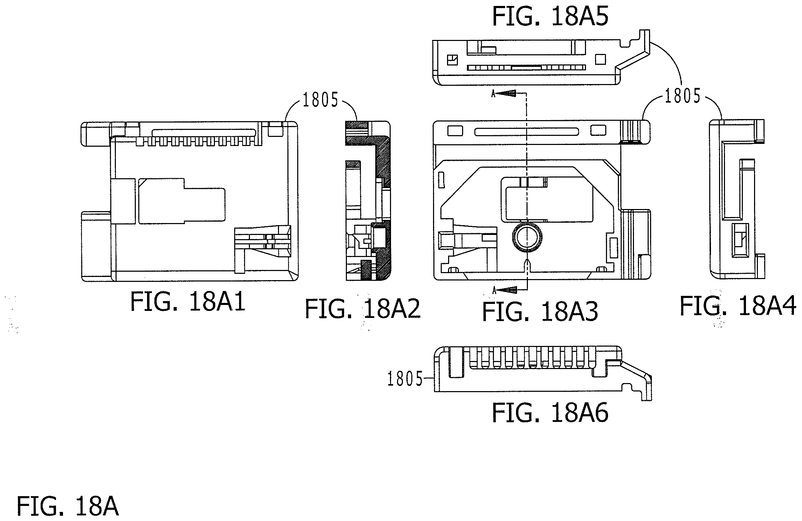

The right side of the apparatus with a push-button locking mechanism is shown in FIG. 5B. The right side comprises a right mount 1805 that is mechanically coupled to the right side strap 1505, a rack 800 that is slidably coupled to the right mount 1805, and a right cover 1005 that is affixed to the right mount 1805 such that the rack 800 is located between the right mount 1805 and the right cover 1005. As described with reference to the left cover 1010, the right cover 1005 in this embodiment is interchangeable with the right cover 1005 of the embodiment without a locking mechanism. Orthogonal views of the right mount 1805 are shown in FIGS. 18A1, 18A2, 18A3, 18A4, and 18A5 (collectively FIG. 18A). In FIG. 5B, a right slide lock 905 and a pinioned dial 710 are located between the right mount 1705 and the right cover 1005. Again, the pinioned dial 710 is rotationally coupled to the right mount 1705 and positioned in such a way as to permit a rack-and-pinion movement between the pinioned dial 710 and the rack 800. The push button 1910, when locked, prevents the rack 800 from sliding.

Providing dual front straps (as shown in FIGS. 1 and 2) and locking mechanisms (as shown in FIGS. 4 and 5), greater security and comfort are provided to the user, thereby improving safety.

Turning to another embodiment, FIGS. 21 through 33 show various embodiments that attach to a fabric, such as a side panel of a baseball cap or other flexible non-rigid head covering. FIG. 21 is a diagram showing one embodiment in which a welding mask 2120 is used in conjunction with a baseball cap 2110 (secured by a knob 110) in such a way that the angle of the welding mask 2120 can be adjusted on each side of the baseball cap 2110. It should be appreciated that, although a baseball cap is shown and described, the head covering can be any flexible non-rigid head covering and is not limited to baseball caps.

Unlike the embodiments shown with reference to FIGS. 1 through 20, the embodiment of FIG. 21 provides attachments so that a user is able to use the head gear in conjunction with a user's own baseball cap or other flexible non-rigid head covering, thereby increasing comfort for the user. With this in mind, the various components that comprise the head gear shown in FIG. 21 are described with reference to FIGS. 22A through 33D.

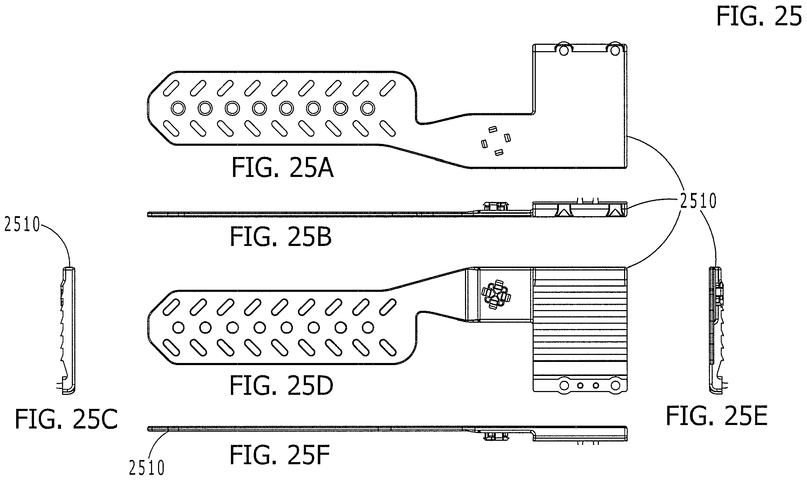

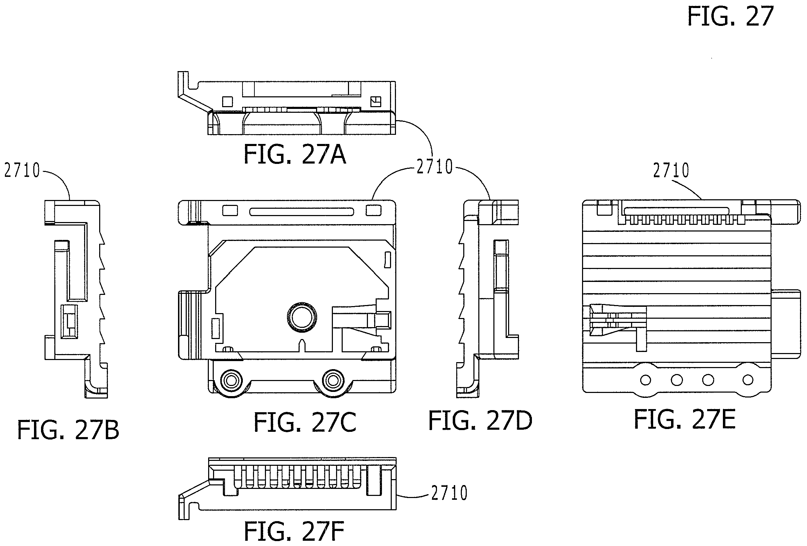

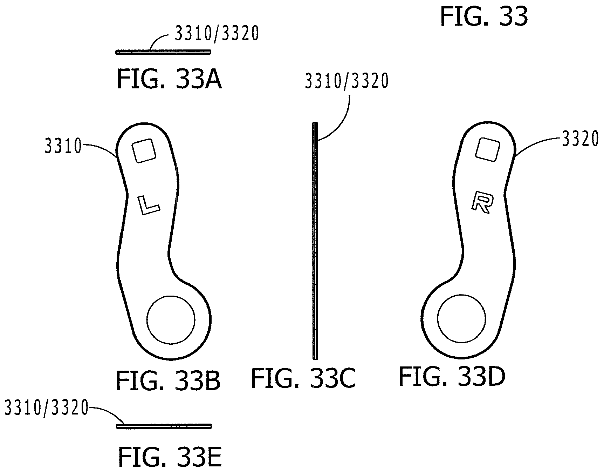

FIGS. 22A and 22B are diagrams showing embodiments of a right side (FIG. 22A) and a left side (FIG. 22B) of a protective head gear without a rack lock, which can be used in conjunction with a baseball cap or other flexible non-rigid head covering. As shown in FIG. 22A, the right side of the head gear comprises a back strap 210, which is pivotally connected to a right back-strap connector 3310. The right back-strap connector 3310 is attached to a right inner clip 2510. A right outer clip 2710 is affixed to the right inner clip 2510 with various screws or other known mounting mechanisms. Within the right outer clip 2710 are a pinioned dial 710 and a right slide lock 905, which are secured to the right outer clip 2710 using the right cover 1005. A rack 800 with indicator pin 820 is slidably coupled to the right outer clip 2710, and a mount is rotatably connected to the rack 800 using a bearing 610 and a knob 110. Orthogonal views of the right back strap connector 3310 are shown in detail with reference to FIGS. 33A, 33C, 33D, and 33E. Orthogonal views of the right inner clip 2510 (for use without a rack lock) are shown in detail with reference to FIGS. 25A, 25B, 25C, 25D, 25E, and 25F (collectively, FIG. 25). Orthogonal views of the right outer clip 2710 (for use without a rack lock) are shown in detail with reference to FIGS. 27A, 27B, 27C, 27D, 27E, and 27F (collectively, FIG. 27).

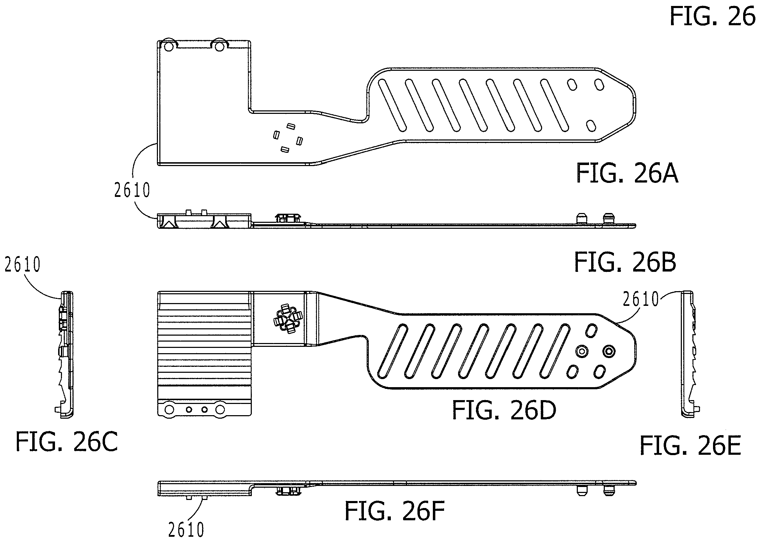

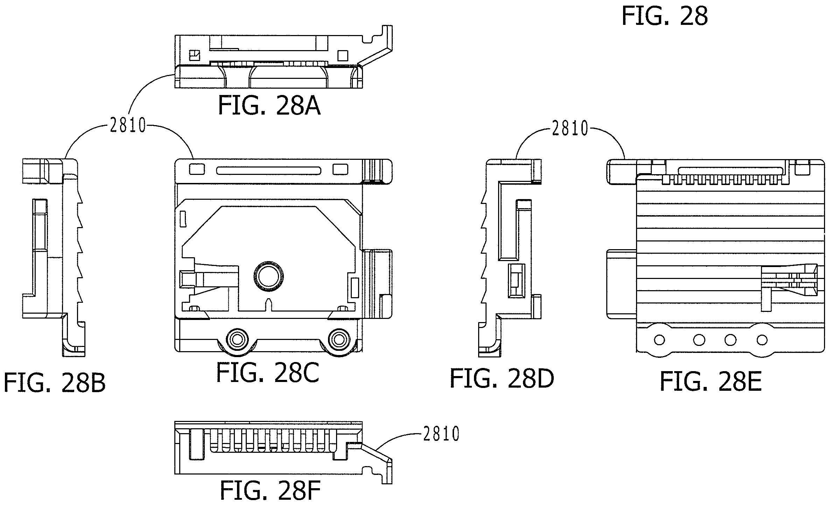

The left side of the head gear (without a rack lock), as shown in FIG. 22B, comprises a back strap 210, which is pivotally connected to a left back-strap connector 3320. The left back-strap connector 3320 is attached to a left inner clip 2610. A left outer clip 2810 is affixed to the left inner clip 2610 with various screws or other known mounting mechanisms. Within the left outer clip 2810 are a pinioned dial 710 and a left slide lock 910, which are secured to the left outer clip 2810 using the left cover 1010. A rack 800 with indicator pin 820 is slidably coupled to the left outer clip 2810, and the mount is rotatably connected to the rack 800 using a bearing 610 and a knob 110. At bottom, the embodiment of FIG. 22B is a complement of the embodiment of FIG. 22A. Orthogonal views of the left back strap connector 3320 are shown in detail with reference to FIGS. 33A, 33B, 33C, and 33E. Orthogonal views of the left inner clip 2610 (for use without a rack lock) are shown in detail with reference to FIGS. 26A, 26B, 26C, 26D, 26E, and 26F (collectively, FIG. 26). Orthogonal views of the left outer clip 2810 (for use without a rack lock) are shown in detail with reference to FIGS. 28A, 28B, 28C, 28D, 28E, and 28F (collectively, FIG. 28).

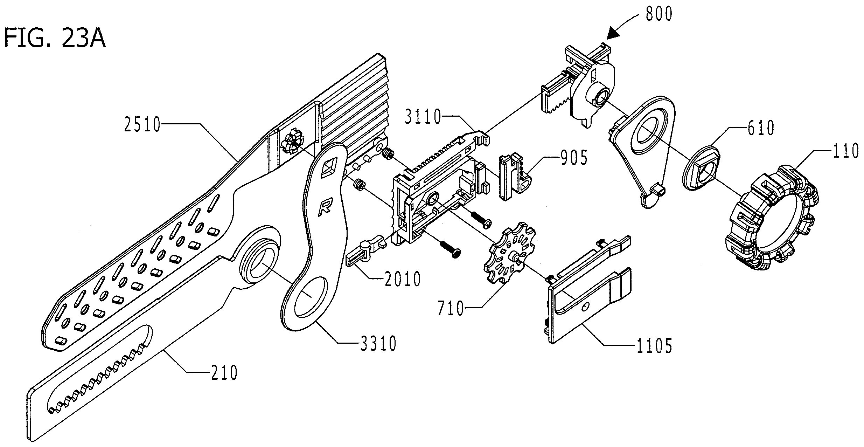

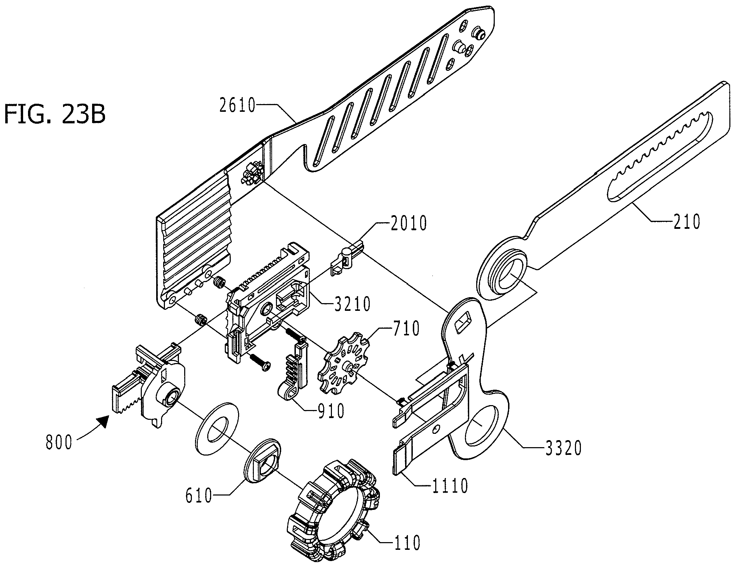

FIGS. 23A and 23B are diagrams showing other embodiments of a right side (FIG. 23A) and a left side (FIG. 23B) of a protective head gear with a paddle lock. The right side of the head gear with the paddle lock comprises a back strap 210, which is pivotally connected to a right back-strap connector 3310. The right back-strap connector 3310 is attached to a right inner clip 2510. Insofar as the back strap 210, the right back-strap connector 3310, and the right inner clip 2510 are described above, further discussion of these components is omitted with reference to FIG. 23A.

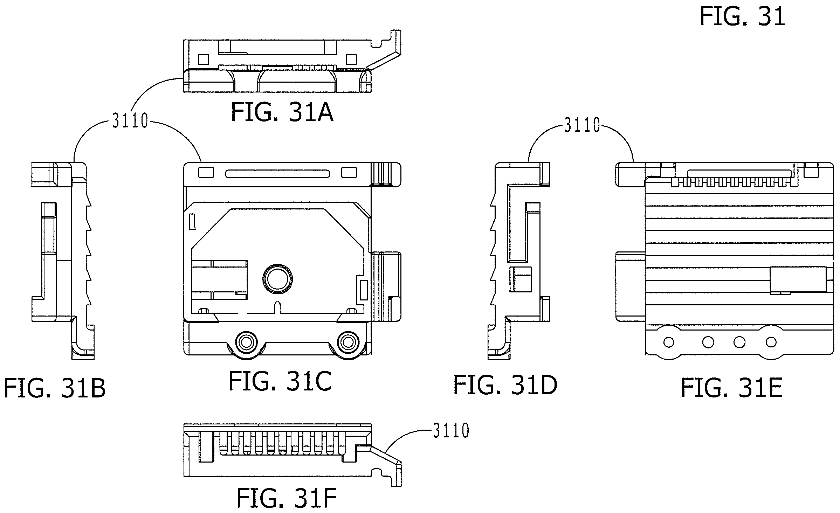

A right outer clip 3110 (for use with a paddle lock) is affixed to the right inner clip 2510 with various screws or other known mounting mechanisms. Within the right outer clip 3110 are a pinioned dial 710, a right slide lock 905, and a paddle 2010, which are secured to the right outer clip 3110 using the right cover 1105. A rack 800 with indicator pin 820 is slidably coupled to the right outer clip 3110, and a mount is rotatably connected to the rack 800 using a bearing 610 and a knob 110. The paddle 2010 pivots to lock and unlock the dial 710, thereby controlling movement of the rack 800. Orthogonal views of the right outer clip 3110 (for use with a paddle lock) are shown in detail with reference to FIGS. 31A, 31B, 31C, 31D, 31E, and 31F (collectively, FIG. 31).

Similar to the right side (FIG. 23A), the left side of the head gear (FIG. 23B) with the paddle lock comprises a back strap 210, which is pivotally connected to a left back-strap connector 3320. The left back-strap connector 3320 is attached to a left inner clip 2610. Insofar as the back strap 210, the left back-strap connector 3320, and the left inner clip 2610 are described above, further discussion of these components is omitted with reference to FIG. 23B.

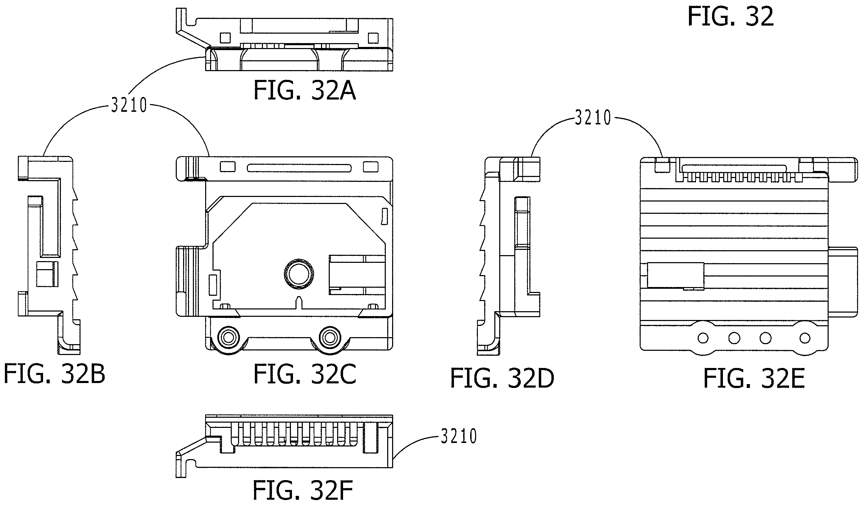

A left outer clip 3210 (for use with a paddle lock) is affixed to the left inner clip 2610 with various screws or other known mounting mechanisms. Within the left outer clip 3210 are a pinioned dial 710, a left slide lock 910, and a paddle 2010, which are secured to the left outer clip 3210 using the left cover 1110. A rack 800 with indicator pin 820 is slidably coupled to the left outer clip 3210, and a mount is rotatably connected to the rack 800 using a bearing 610 and a knob 110. The paddle 2010 pivots to lock and unlock the dial 710, thereby controlling movement of the rack 800. Orthogonal views of the left outer clip 3210 (for use with a paddle lock) are shown in detail with reference to FIGS. 32A, 32B, 32C, 32D, 32E, and 32F (collectively, FIG. 32).

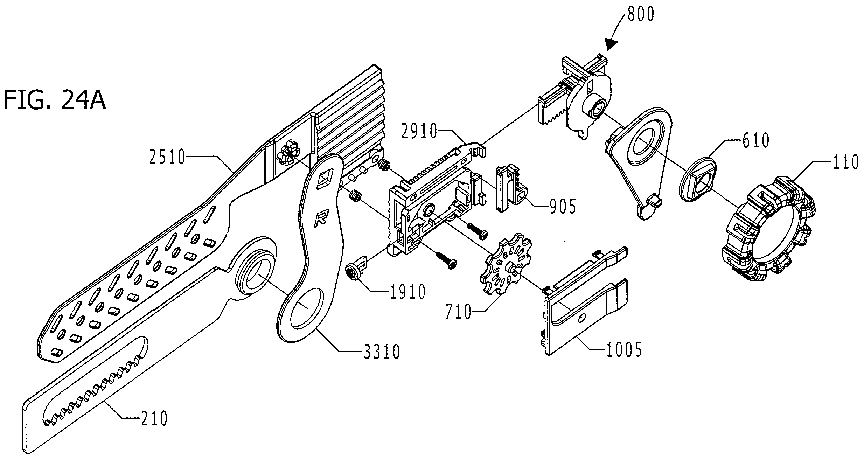

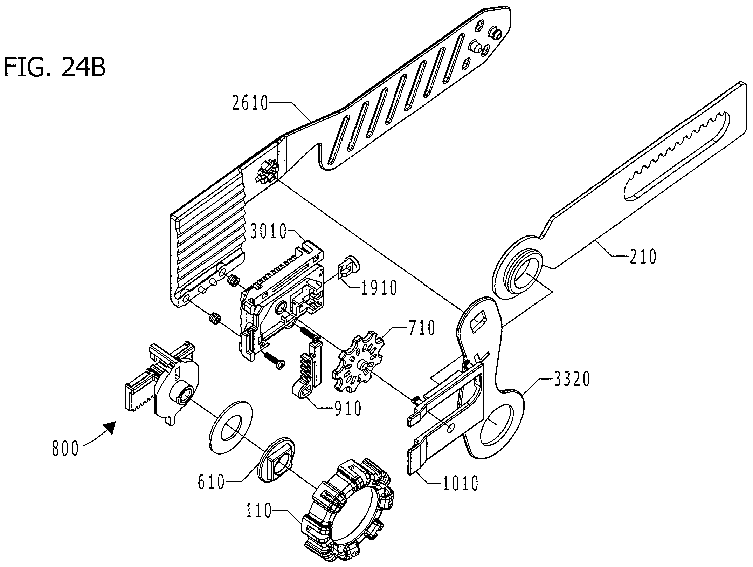

FIGS. 24A and 24B are diagrams showing other embodiments of a right side (FIG. 24A) and a left side (FIG. 24B) of a protective head gear with a push-button lock. The right side of the head gear with the push-button lock comprises a back strap 210, which is pivotally connected to a right back-strap connector 3310. The right back-strap connector 3310 is attached to a right inner clip 2510. Insofar as the back strap 210, the right back-strap connector 3310, and the right inner clip 2510 are described above, further discussion of these components is omitted with reference to FIG. 24A.

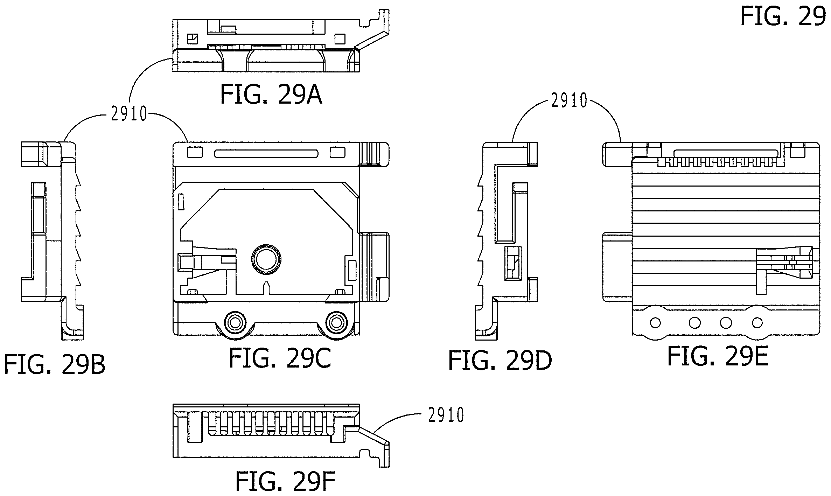

A right outer clip 2910 (for use with a push-button lock) is affixed to the right inner clip 2510 with various screws or other known mounting mechanisms. Within the right outer clip 2910 are a pinioned dial 710, a right slide lock 905, and a push button 1910, which are secured to the right outer clip 2910 using the right cover 1005. A rack 800 with indicator pin 820 is slidably coupled to the right outer clip 2910, and a mount is rotatably connected to the rack 800 using a bearing 610 and a knob 110. The push button 1910 toggles or depresses to lock and unlock the dial 710, which in turn controls movement of the rack 800. Orthogonal views of the right outer clip 2910 (for use with a push-button lock) are shown in detail with reference to FIGS. 29A, 29B, 29C, 29D, 29E, and 29F (collectively, FIG. 29).

Similar to the right side (FIG. 24A), the left side of the head gear (FIG. 24B) with the push-button lock comprises a back strap 210, which is pivotally connected to a left back-strap connector 3300. The left back-strap connector 3300 is attached to a left inner clip 2610. Insofar as the back strap 210, the left back-strap connector 3300, and the left inner clip 2610 are described above, further discussion of these components is omitted with reference to FIG. 24B.

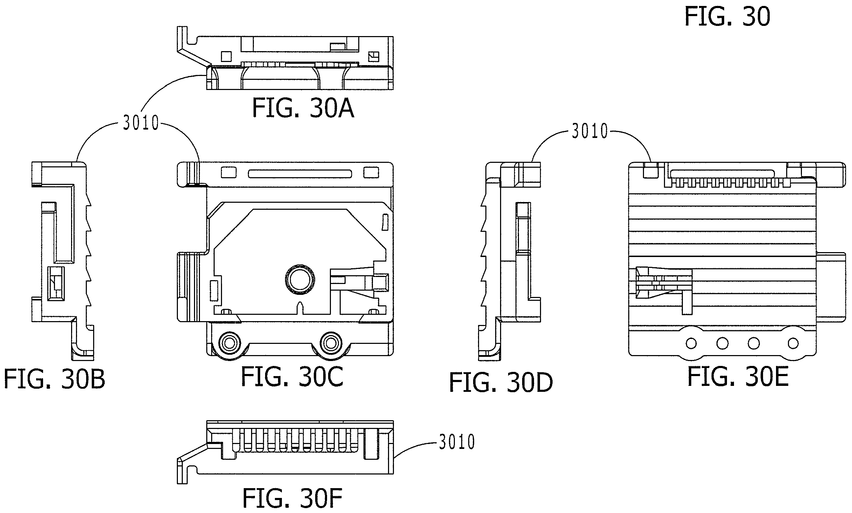

A left outer clip 3010 (for use with a push-button lock) is affixed to the left inner clip 2610 with various screws or other known mounting mechanisms. Within the left outer clip 3010 are a pinioned dial 710, a left slide lock 910, and a push-button 1910, which are secured to the left outer clip 3010 using the left cover 1010. A rack 800 with indicator pin 820 is slidably coupled to the left outer clip 3010, and a mount is rotatably connected to the rack 800 using a bearing 610 and a knob 110. The push-button 1910 toggles or depresses to lock and unlock the dial 710, thereby controlling movement of the rack 800. Orthogonal views of the left outer clip 3010 (for use with a push-button lock) are shown in detail with reference to FIGS. 30A, 30B, 30C, 30D, 30E, and 30F (collectively, FIG. 30).

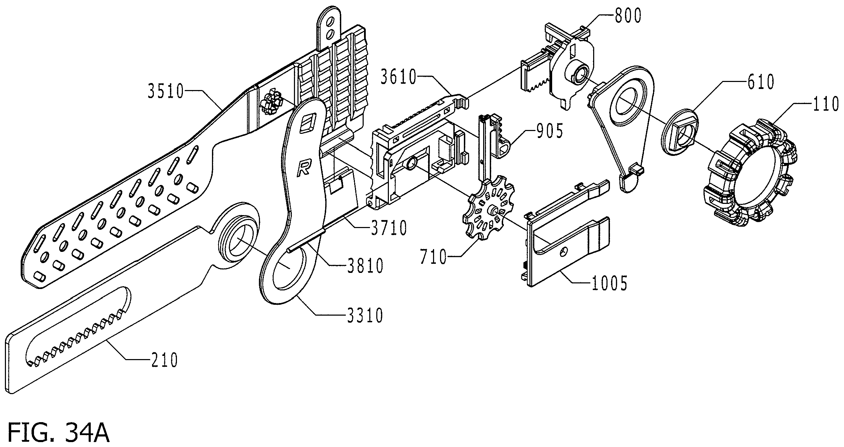

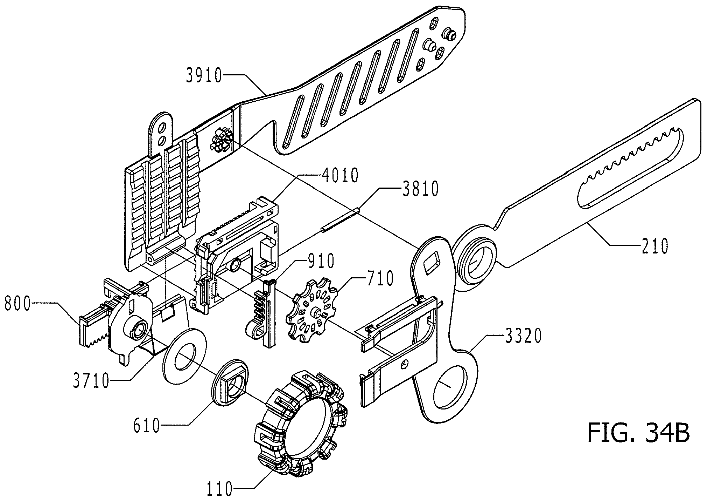

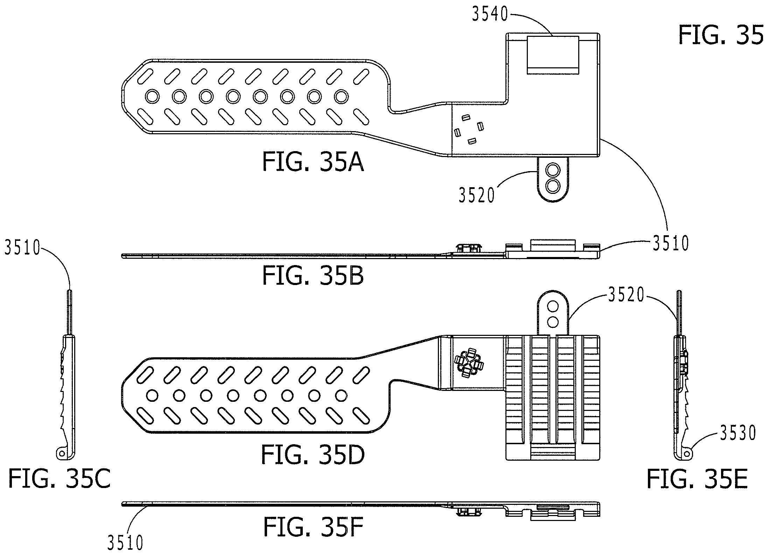

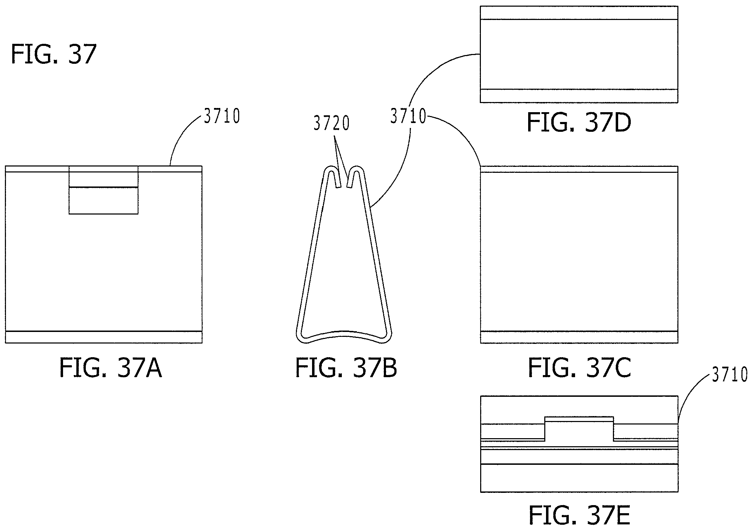

FIGS. 34A and 34B are diagrams showing a right side (FIG. 34A) and a left side (FIG. 34B) of another embodiment of a protective head gear. Specifically, the embodiment of FIGS. 34A and 34B are intended for use with a non-rigid flexible head covering, such as a baseball cap. As shown in FIG. 34A, the right side of the head gear comprises a back strap 210, which is pivotally connected to a right back-strap connector 3310. The right back-strap connector 3310 is attached to a right inner clip 3510 for use with a spring clip 3710. Orthogonal views of the right inner clip 3510 are shown in greater detail with reference to FIGS. 35A, 35B, 35C, 35D, 35E, and 35F (collectively, FIG. 35) and orthogonal views of the spring clip 3710 are shown in greater detail with reference to FIGS. 37A, 37B, 37C, 37D, and 37E (collectively, FIG. 37). Notably, as shown in FIG. 35, the right inner clip 3510 comprises an adjustment point 3510 for an available top strap (not shown in FIG. 35) and a slot 3540 for the spring clip 3710. Additionally, the right inner clip 3510 comprises an inner clip hinge point 3530, which is described in greater detail below. Insofar as the back strap 210 and the right back-strap connector 3310 are described above, further discussion of these components is omitted with reference to FIG. 34A.

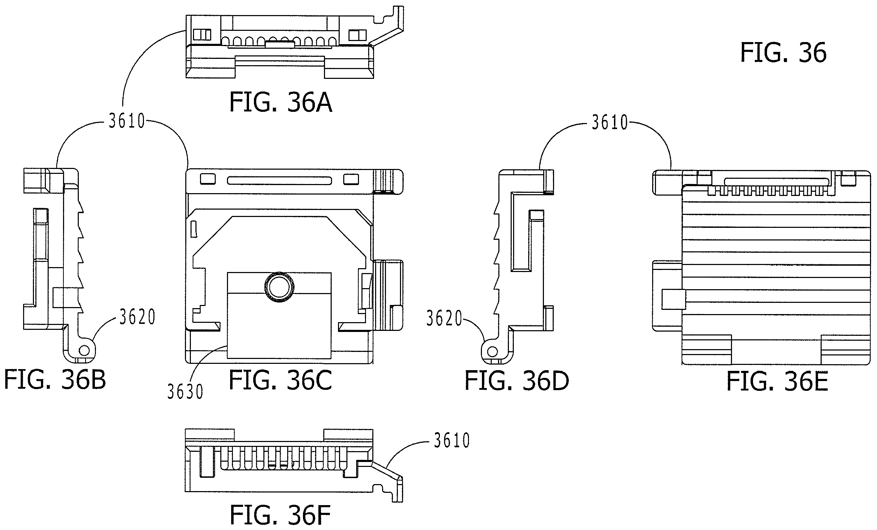

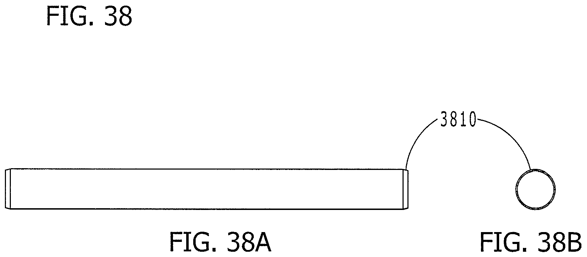

Continuing with FIG. 34A, a right outer clip 3610 is connected to the right inner clip 3510 via a pin 3810. Orthogonal views of the pin 3810 are shown with reference to FIGS. 38A and 38B (collectively, FIG. 38), while orthogonal views of the right outer clip 3610 are shown with reference to FIGS. 36A, 36B, 36C, 36D, 36E, and 36F (collectively, FIG. 36). Notably, the right outer clip 3610 comprises an outer clip hinge point 3620, which pivotally mates with the inner clip hinge point 3530 using the pin 3810. Additionally, the right outer clip 3610 comprises a slot 3630 for the spring clip 3710.

Thus, in operation for one embodiment, an edge of a fabric from a non-rigid head covering (e.g., baseball cap, etc.) is placed in the angle that is formed between the right outer clip 3510 and the right inner clip 3610 at the pin 3810. Once the fabric is clamped between the right outer clip 3510 and the right inner clip 3610, the spring clip 3710 is slid onto the slots 3540, 3630, thereby securing the right clip 3510, 3610 onto the fabric. As shown in FIG. 37, the spring clip 3710 comprises locking teeth 3710 to hold the spring clip 3710 in place.

In operation for another embodiment, the spring clip 3710 is slid into the slots 3540, 3630 prior to installing the right clip 3510, 3610 onto the fabric, thereby providing a variable clamping force that is dependent on the flexibility of the spring clip 3710. This permits the user to adjust, remove, or install the right clip 3510, 3610 onto an edge of a fabric by bending the spring clip 3710 at the hinge point 3610, 3620.

Additionally, as shown in FIG. 34A, within the right outer clip 3610 are a pinioned dial 710 and a right slide lock 905, which are secured to the right outer clip 3610 using a right cover 1005. A rack 800 with indicator pin 820 is slidably coupled to the right outer clip 3610, and a mount is rotatably connected to the rack 800 using a bearing 610 and a knob 110. Insofar as the pinioned dial 710, right slide lock 905, rack 800, indicator pin 820, bearing 610, and knob 110 have been described above, further discussion of those components is omitted with reference to FIG. 34A.

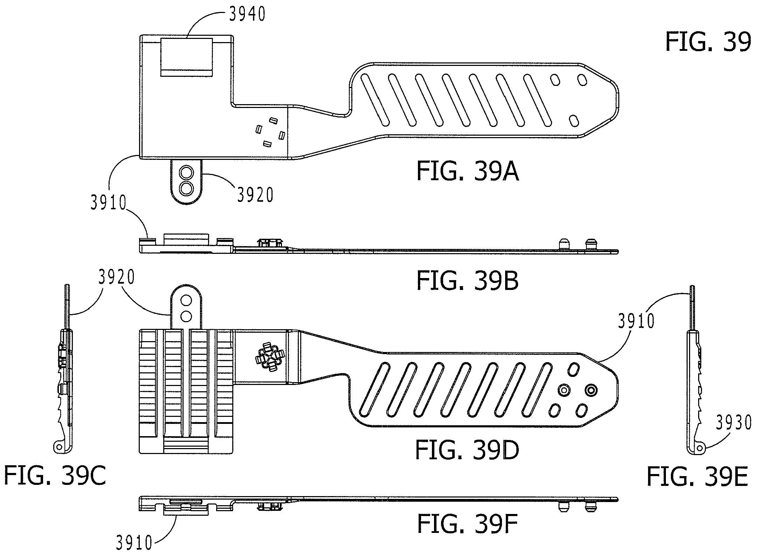

Turning to FIG. 34B, the left side of the head gear comprises a back strap 210, which is pivotally connected to a left back-strap connector 3320. The left back-strap connector 3320 is attached to a left inner clip 3910 that, similar to the right inner clip 3510, is intended for use with a spring clip 3710. Orthogonal views of the left inner clip 3910 are shown in greater detail with reference to FIGS. 39A, 39B, 39C, 39D, 39E, and 39F (collectively, FIG. 39). Notably, as shown in FIG. 39, the left inner clip 3910 comprises an adjustment point 3910 for an available top strap (not shown in FIG. 39) and an inner clip hinge point 3930. Insofar as the back strap 210 and the left back-strap connector 3320 are described above, further discussion of these components is omitted with reference to FIG. 34B.

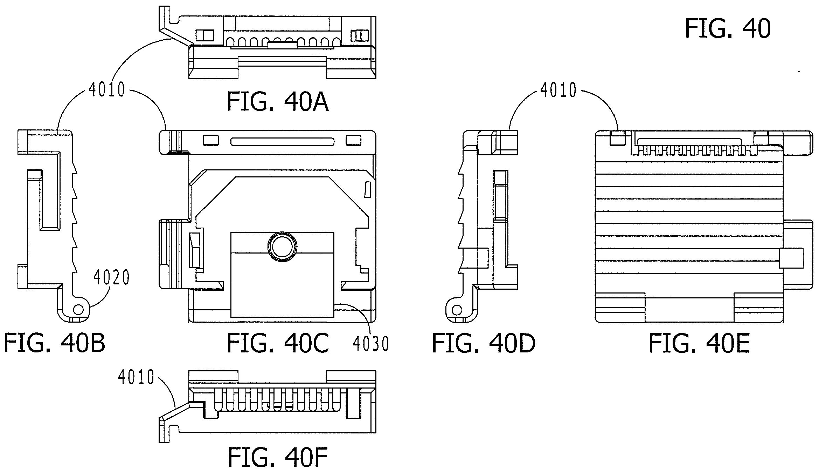

Continuing with FIG. 34B, a left outer clip 4010 is connected to the left inner clip 3910 via a pin 3810. Orthogonal views of the left outer clip 4010 are shown with reference to FIGS. 40A, 40B, 40C, 40D, 40E, and 40F (collectively, FIG. 40). Notably, the left outer clip 4010 comprises an outer clip hinge point 4020, which pivotally mates with the inner clip hinge point 3930 using the pin 3810. Additionally, the left outer clip 4010 comprises a slot 4030 for the spring clip 3710.

Thus, in operation for one embodiment, for the left side (similar to the right side) an edge of a fabric from a non-rigid head covering (e.g., baseball cap, etc.) is placed in the angle that is formed between the left outer clip 3910 and the left inner clip 4010 at the pin 3810. Once the fabric is clamped between the left outer clip 3910 and the left inner clip 4010, the spring clip 3710 is slid onto the slot 4030, thereby securing the left clip 3910, 4010 onto the fabric.

In operation for another embodiment, the spring clip 3710 is slid into the slot 4030 prior to installing the left clip 3910, 4010 onto the fabric, thereby providing a variable clamping force that is dependent on the flexibility of the spring clip 3710. Similar to the right clip 3510, 3610, the flexibility of the spring clip 3710 in this embodiment permits the user to adjust, remove, or install the left clip 3910, 4010 onto the edge of the fabric by bending the spring clip 3710 at the hinge point 3930, 4020.

Additionally, as shown in FIG. 34B, within the left outer clip 4010 are a pinioned dial 710 and a left slide lock 910, which are secured to the left outer clip 4010 using a left cover 1010. A rack 800 with indicator pin 820 is slidably coupled to the left outer clip 4010, and a mount is rotatably connected to the rack 800 using a bearing 610 and a knob 110. Insofar as the pinioned dial 710, left slide lock 910, rack 800, indicator pin 820, bearing 610, and knob 110 have been described above, further discussion of those components is omitted with reference to FIG. 34B.

Providing attachments (as shown with reference to FIGS. 21 through 40F), so that a user is able to use the head gear in conjunction with a user's own baseball cap or other flexible non-rigid head covering, increases comfort for the user. The increased comfort provides less distraction to the user, thereby increasing safety. Furthermore, by providing various locking mechanisms that prevent the rack 800 from moving (when locked), the embodiments shown in FIGS. 1 through 40F provide greater security and, therefore, improve safety of the protective head gear.

Although exemplary embodiments have been shown and described, it will be clear to those of ordinary skill in the art that a number of changes, modifications, or alterations to the disclosure as described may be made. For example, while various embodiments are described with reference to safety equipment and protective head gear in work environments, it should be appreciated that the adapter can be used in different environments, such as, for example, athletic or sports environments. Also, it should be appreciated that the left side and the right side are interchangeable, since left and right are dependent on the perspective (e.g., whether facing toward the operator, or facing away from the operator). All such changes, modifications, and alterations should therefore be seen as within the scope of the disclosure, as defined by the claims.

## Claims

1. An apparatus (100), comprising:
a left side strap (1510);
a left pivot point located on the left side strap;
a right side strap (1505);
a right pivot point located on the right side strap;
a lower front strap (1310);
a lower left attachment point located on the lower front strap, the lower left attachment point being pivotally connected to the left pivot point;
a lower right attachment point located on the lower front strap, the lower right attachment point being pivotally connected to the right pivot point;
an upper front strap (1410);
an upper left attachment point located on the upper front strap, the upper left attachment point being pivotally connected to the left pivot point; and
an upper right attachment point located on the upper front strap, the upper right attachment point being pivotally connected to the right pivot point,
**characterized in that** the lower front strap (1310) and the upper front strap (1410) are independently movable, so the two front straps (1310, 1410) can be positioned independently at different angles.

2. The apparatus of claim 1, further comprising:
a left mount (1610) mechanically coupled to the left side strap;
a left rack slidably coupled to the left mount;
a right mount (1605) mechanically coupled to the right side strap; and
a right rack slidably couple to the right mount.

3. The apparatus of claim 2, further comprising:
a left cover (1110) mechanically coupled to the left mount, the left rack being located between the left mount and the left cover; and
a right cover (1105) mechanically coupled to the right mount, the right rack being located between the right mount and the right cover.

4. The apparatus of claim 3, further comprising:
a left slide lock (910) located between the left mount and the left cover; and
a right slide lock (905) located between the right mount and the right cover.

5. The apparatus of claim 3, further comprising:
left teeth located on the left rack;
a left pinioned dial rotationally coupled to the left mount, the left pinioned dial engaging with the left teeth for a left rack-and-pinion movement;
right teeth located on the right rack; and
a right pinioned dial rotationally coupled to the right mount, the right pinioned dial engaging with the right teeth for a right rack-and-pinion movement.

6. The apparatus of claim 3, further comprising a push-button lock, the push-button lock being one selected from the group consisting of:
a left push-button lock located on the left mount, the left push-button lock for selectively preventing movement of the left rack; and
a right push-button lock located on the right mount, the right push-button lock for selectively preventing movement of the right rack.

7. The apparatus of claim 3, further comprising a paddle lock, the paddle lock being one selected from the group consisting of:
a left paddle lock located on the left mount, the left paddle lock for selectively preventing movement of the left rack; and
a right paddle lock located on the right mount, the right paddle lock for selectively preventing movement of the right rack.

8. The apparatus of claim 1, further comprising:
a left top attachment point located on the left side strap;
a left top-strap attachment pivotally coupled to the left top attachment point;
a right top attachment point located on the right side strap; and
a right top-strap attachment pivotally coupled to the right top attachment point.

9. The apparatus of claim 1, further comprising:
a rear strap pivotally coupled to the left side strap, the rear strap further pivotally coupled to the right side strap.

10. The apparatus of claim 1, further comprising:
a right knob coupled to the right rack.

11. The apparatus of claim 1, further comprising:
a left knob coupled to the left rack.

## Patentansprüche

1. Eine Vorrichtung (100), die folgende Merkmale aufweist:
einen linken Seitenriemen (1510);
einen auf dem linken Seitenriemen befindlichen linken Drehpunkt;
einen rechten Seitenriemen (1505);
einen auf dem rechten Seitenriemen befindlichen rechten Drehpunkt;
einen unteren vorderen Riemen (1310);
einen unteren linken Befestigungspunkt, der sich an dem unteren vorderen Riemen befindet, wobei der untere linke Befestigungspunkt drehbar mit dem linken Drehpunkt verbunden ist;
einen unteren rechten Befestigungspunkt, der sich an dem unteren vorderen Riemen befindet, wobei der untere rechte Befestigungspunkt drehbar mit dem rechten Drehpunkt verbunden ist;
einen oberen vorderen Riemen (1410);
einen oberen linken Befestigungspunkt, der sich an dem oberen vorderen Riemen befindet, wobei der obere linke Befestigungspunkt drehbar mit dem linken Drehpunkt verbunden ist; und
einen oberen rechten Befestigungspunkt, der sich an dem oberen vorderen Riemen befindet, wobei der obere rechte Befestigungspunkt drehbar mit dem rechten Drehpunkt verbunden ist;
**dadurch gekennzeichnet, dass** der untere vordere Riemen (1310) und der obere vordere Riemen (1410) unabhängig voneinander bewegbar sind, so dass die zwei vorderen Riemen (1310, 1410) unabhängig voneinander in unterschiedlichen Winkeln positioniert werden können.

2. Die Vorrichtung gemäß Anspruch 1, die ferner folgende Merkmale aufweist:
eine mit dem linken Seitenriemen mechanisch gekoppelte linke Halterung (1610);
eine mit der linken Halterung schiebbar gekoppelte linke Zahnstange;
eine mit dem rechten Seitenriemen mechanisch gekoppelte rechte Halterung (1605); und
eine mit der rechten Halterung schiebbar gekoppelte rechte Zahnstange.

3. Die Vorrichtung gemäß Anspruch 2, die ferner folgende Merkmale aufweist:
eine linke Abdeckung (1110), die mit der linken Halterung mechanisch gekoppelt ist, wobei sich die linke Zahnstange zwischen der linken Halterung und der linken Abdeckung befindet; und
eine rechte Abdeckung (1105), die mit der rechten Halterung mechanisch gekoppelt ist, wobei sich die rechte Zahnstande zwischen der rechten Halterung und der rechten Abdeckung befindet.

4. Die Vorrichtung gemäß Anspruch 3, die ferner folgende Merkmale aufweist:
eine linke Schieberraste (910), die sich zwischen der linken Halterung und der linken Abdeckung befindet; und
eine rechte Schieberraste (905), die sich zwischen der rechten Halterung und der rechten Abdeckung befindet.

5. Die Vorrichtung gemäß Anspruch 3, die ferner folgende Merkmale aufweist:
auf der linken Zahnstange befindliche linke Zähne;
eine linke Ritzelscheibe, die drehbar mit der linken Halterung gekoppelt ist, wobei die linke Ritzelscheibe mit den linken Zähnen zum Zweck einer Zahnstangen-Linksbewegung in Eingriff steht;
auf der rechten Zahnstange befindliche rechte Zähne; und
eine rechte Ritzelscheibe, die drehbar mit der rechten Halterung gekoppelt ist, wobei die rechte Ritzelscheibe mit den rechten Zähnen zum Zweck einer Zahnstangen-Rechtsbewegung in Eingriff steht.

6. Die Vorrichtung gemäß Anspruch 3, die ferner eine Druckknopfverriegelung aufweist, wobei die Druckknopfverriegelung aus der aus Folgenden bestehenden Gruppe ausgewählt ist:
einer sich an der linken Halterung befindlichen linken Druckknopfverriegelung, wobei die linke Druckknopfverriegelung zum selektiven Verhindern einer Bewegung der linken Zahnstange dient; und
einer sich an der rechten Halterung befindlichen rechten Druckknopfverriegelung, wobei die rechte Druckknopfverriegelung zum selektiven Verhindern einer Bewegung der rechten Zahnstange dient.

7. Die Vorrichtung gemäß Anspruch 3, die ferner eine Kipphebelverriegelung aufweist, wobei die Kipphebelverriegelung aus der aus Folgenden bestehenden Gruppe ausgewählt ist:
einer an der linken Halterung befindliche linke Kippschalterverriegelung, wobei die linke Kippschalterverriegelung einem selektiven Verhindern einer Bewegung der linken Zahnstange dient; und
einer an der rechten Halterung befindliche rechte Kippschalterverriegelung, wobei die rechte Kippschalterverriegelung einem selektiven Verhindern einer Bewegung der rechten Zahnstange dient.

8. Die Vorrichtung gemäß Anspruch 1, die ferner folgende Merkmale aufweist:
einen linken oberen Befestigungspunkt, der sich an dem linken Seitenriemen befindet;
eine linke Oberer-Riemen-Befestigung, die drehbar mit dem linken oberen Befestigungspunkt gekoppelt ist;
einen rechten oberen Befestigungspunkt, der sich an dem rechten Seitenriemen befindet; und
eine rechte Oberer-Riemen-Befestigung, die drehbar mit dem rechten oberen Befestigungspunkt gekoppelt ist.

9. Die Vorrichtung gemäß Anspruch 1, die ferner folgendes Merkmal aufweist:
einen hinteren Riemen, der drehbar mit dem linken Seitenriemen gekoppelt ist, wobei der hintere Riemen ferner drehbar mit dem rechten Seitenriemen gekoppelt ist.

10. Die Vorrichtung gemäß Anspruch 1, die ferner folgendes Merkmal aufweist:
einen mit der rechten Zahnstange gekoppelten rechten Drehknopf.

11. Die Vorrichtung gemäß Anspruch 1, die ferner folgendes Merkmal aufweist:
einen mit der linken Zahnstange gekoppelten linken Drehknopf.

## Revendications

1. Appareil (100), comprenant:
une sangle latérale gauche (1510);
un point de pivotement gauche situé sur la sangle latérale gauche;
une sangle latérale droite (1505);
un point de pivotement droit situé sur la sangle latérale droite;
une sangle avant inférieure (1310);
un point de fixation gauche inférieur situé sur la sangle avant inférieure, le point de fixation gauche inférieur étant connecté de manière pivotante au point de pivotement gauche;
un point de fixation droit inférieur situé sur la sangle avant inférieure, le point de fixation droit inférieur étant connecté de manière pivotante au point de pivotement droit;
une sangle avant supérieure (1410;
un point de fixation gauche supérieur situé sur la sangle avant supérieure, le point de fixation gauche supérieur étant connecté de manière pivotante au point de pivotement gauche; et
un point de fixation droit supérieur situé sur la sangle avant supérieure, le point de fixation droit supérieur étant connecté de manière pivotante au point de pivotement droit,
**caractérisé par le fait que** la sangle avant inférieure (1310) et la sangle avant supérieure (1410) sont déplaçables indépendamment l'une de l'autre, de sorte que les deux sangles avant (1310, 1410) puissent être positionnées indépendamment selon des angles différents.

2. Appareil selon la revendication 1, comprenant par ailleurs:
un support gauche (1610) couplé mécaniquement à la sangle latérale gauche;
une crémaillère gauche couplée de manière coulissante au support gauche;
un support droit (1605) couplé mécaniquement à la sangle latérale droite; et
une crémaillère droite couplée de manière coulissante au support droit.

3. Appareil selon la revendication 2, comprenant par ailleurs:
un couvercle gauche (1110) couplé mécaniquement au support gauche, la crémaillère gauche étant située entre le support gauche et le couvercle gauche; et
un couvercle droit (1105) couplé mécaniquement au support droit, la crémaillère droite étant située entre le support droit et le couvercle droit.

4. Appareil selon la revendication 3, comprenant par ailleurs:
un verrou coulissant gauche (910) situé entre le support gauche et le couvercle gauche; et
un verrou coulissant droit (905) situé entre le support droit et le couvercle droit.

5. Appareil selon la revendication 3, comprenant par ailleurs:
des dents gauches situées sur la crémaillère gauche;
un cadran à pignon gauche couplé en rotation au support gauche, le cadran à pignon gauche engrenant avec les dents gauches pour un mouvement par crémaillère gauche;
des dents droites situées sur le support droit; et
un cadran à pignon droit couplé en rotation au support droit, le cadran à pignon droit engrenant avec les dents droites pour un mouvement par crémaillère droit.

6. Appareil selon la revendication 3, comprenant par ailleurs un verrou à bouton-poussoir, le verrou à bouton-poussoir étant l'un sélectionné parmi le groupe composé de:
un verrou à bouton-poussoir gauche situé sur le support gauche, le verrou à bouton-poussoir gauche étant destiné à empêcher de manière sélective le mouvement de la crémaillère gauche; et
un verrou à bouton-poussoir droit situé sur le support droit, le verrou à bouton-poussoir droit étant destiné à empêcher de manière sélective le mouvement de la crémaillère droite.

7. Appareil selon la revendication 3, comprenant par ailleurs un verrou à palette, le verrou à palette étant l'un sélectionné parmi le groupe composé de:
un verrou à palette gauche situé sur le support gauche, le verrou à palette gauche étant destiné à empêcher de manière sélective le mouvement de la crémaillère gauche; et
un verrou à palette droit situé sur le support droit, le verrou à palette droit étant destiné à empêcher de manière sélective le mouvement de la crémaillère droite.

8. Appareil selon la revendication 1, comprenant par ailleurs:
un point de fixation supérieur gauche situé sur la sangle latérale gauche;
une fixation de sangle supérieure gauche couplée de manière pivotante au point de fixation supérieur gauche;
un point de fixation supérieur droit situé sur la sangle latérale droite; et
une fixation de sangle supérieure droite couplée de manière pivotante au point de fixation supérieur droit.

9. Appareil selon la revendication 1, comprenant par ailleurs:
une sangle arrière couplée de manière pivotante à la sangle latérale gauche, la sangle arrière étant par ailleurs couplée de manière pivotante à la sangle latérale droite.

10. Appareil selon la revendication 1, comprenant par ailleurs:
un bouton droit couplé à la crémaillère droite.

11. Appareil selon la revendication 1, comprenant par ailleurs:
un bouton gauche couplé à la crémaillère gauche.
